# EUROPEAN PATENT SPECIFICATION

(11) **EP 2 865 384 B1**
(45) Date of publication and mention of the grant of the patent: **15.08.2018**
(21) Application number: 13807573.4
(22) Date of filing: 21.06.2013
(51) Int. Cl.: A61K 38/00, A61P 29/00, C07K 14/435, C12P 21/02, C12N 15/09, C07K 14/47, G01N 33/68

(54) **AGENT FOR TREATING OR PREVENTING SYSTEMIC INFLAMMATORY RESPONSE SYNDROME**
MITTEL ZUR BEHANDLUNG ODER VORBEUGUNG VON SYSTEMISCHEM ENTZÜNDUNGSREAKTIONSSYNDROM
AGENT DE TRAITEMENT OU DE PRÉVENTION DU SYNDROME INFLAMMATOIRE SYSTÉMIQUE

(30) Priority: 22.06.2012 JP 2012141380
(43) Date of publication of application: 29.04.2015
(73) Proprietor: The University of Tokyo, Bunkyo-ku, Tokyo 113-8654 (JP)
(72) Inventor: HAMAKUBO, Takao, Tokyo 113-8654 (JP); TSUMOTO, Kouhei, Tokyo 113-8654 (JP); DAIGO, Kenji, Tokyo 113-8654 (JP); INOUE, Kenji, Tokyo 113-8421 (JP); YAMAGUCHI, Naotaka, Yamaguchi-shi Yamaguchi 753-0048 (JP); MIZUUCHI, Motoaki, Tsukuba-shi Ibaraki 305-0841 (JP); FUJII, Hiroya, Tsukuba-shi Ibaraki 305-0841 (JP)
(74) Representative: dompatent von Kreisler Selting Werner - Partnerschaft von Patent- und Rechtsanwälten mbB
(86) International application number: PCT/JP2013/067122
(87) International publication number: WO 2013/191280

(56) References cited:
- WO-A1-92/12176
- WO-A1-2005/060988
- WO-A1-2005/107791
- JP-A- 2002 503 642
- JP-A- 2004 512 372
- JP-A- 2004 513 151
- GARLANDA CECILIA ET AL: "Pentraxins at the crossroads between innate immunity, inflammation, matrix deposition, and female fertility", ANNUAL REVIEW OF IMMUNOLOGY, ANNUAL REVIEWS INC, US, vol. 23, 1 January 2005 (2005-01-01), pages 337-366, XP002539429, ISSN: 0732-0582, DOI: 10.1146/ANNUREV.IMMUNOL.23.021704.115756
- DIAS,A.A. ET AL.: 'TSG-14 transgenic mice have improved survival to endotoxemia and to CLP- induced sepsis' JOURNAL OF LEUKOCYTE BIOLOGY vol. 69, no. 6, 2001, pages 928 - 36, XP055182480
- DAIGO,K. ET AL.: 'The proteomic profile of circulating pentraxin 3 (PTX3) complex in sepsis demonstrates the interaction with azurocidin 1 and other components of neutrophil extracellular traps' MOLECULAR AND CELLULAR PROTEOMICS vol. 11, no. 6, January 2012, pages M111. 015073, P.1 - 12, XP055182528
- SAUNERO-NAVA,L. ET AL.: 'Hamster female protein binding to chromatin, histones and DNA' MOLECULAR IMMUNOLOGY vol. 29, no. 7-8, 1992, pages 837 - 45, XP023682633
- DAIGO,K. ET AL.: 'Host-protective effect of circulating pentraxin 3 (PTX3) and complex formation with neutrophil extracellular traps' FRONTIERS IN IMMUNOLOGY vol. 3, December 2012, page 378, XP055182532

### SEQUENCE LISTING

<110> University of Tokyo Juntendo University Perseus Proteomics Inc. JSR Corporation
<120> Therapeutic or prophylactic agent and diagnostic agent for SIRS
<130> 092039
<150> JP 2012-141380
   <151> 2012-06-22
<160> 28
<170> PatentIn version 3.4
<210> 1
   <211> 1908
   <212> DNA
   <213> Artificial
<220>
   <223> Histone H3 fragment
<220>
   <221> CDS
   <222> (117)..(1262)
<400> 1
   catttattaa ggactctctg ctccagcctc tcactctcac tctcctccgc tcaaactcag      60
   ctcacttgag agtctcctcc cgccagctgt ggaaagaact ttgcgtctct ccagca atg     119
                                                                  Met
                                                                  1
   cat ctc ctt gcg att ctg ttt tgt gct ctc tgg tct gca gtg ttg gcc       167
   His Leu Leu Ala Ile Leu Phe Cys Ala Leu Trp Ser Ala Val Leu Ala
               5                   10                  15
   gag aac tcg gat gat tat gat ctc atg tat gtg aat ttg gac aac gaa       215
   Glu Asn Ser Asp Asp Tyr Asp Leu Met Tyr Val Asn Leu Asp Asn Glu
           20                  25                  30
   ata gac aat gga ctc cat ccc act gag gac ccc acg ccg tgc gac tgc       263
   Ile Asp Asn Gly Leu His Pro Thr Glu Asp Pro Thr Pro Cys Asp Cys
       35                  40                  45
   ggt cag gag cac tcg gaa tgg gac aag ctc ttc atc atg ctg gag aac       311
   Gly Gln Glu His Ser Glu Trp Asp Lys Leu Phe Ile Met Leu Glu Asn
   50                  55                  60                  65
   tcg cag atg aga gag cgc atg ctg ctg caa gcc acg gac gac gtc ctg       359
   Ser Gln Met Arg Glu Arg Met Leu Leu Gln Ala Thr Asp Asp Val Leu
                   70                  75                  80
   cgg ggc gag ctg cag agg ctg cgg gag gag ctg ggc cgg ctc gcg gaa       407
   Arg Gly Glu Leu Gln Arg Leu Arg Glu Glu Leu Gly Arg Leu Ala Glu
               85                  90                  95
   agc ctg gcg agg ccg tgc gcg ccg ggg gct ccc gca gag gcc agg ctg       455
   Ser Leu Ala Arg Pro Cys Ala Pro Gly Ala Pro Ala Glu Ala Arg Leu
           100                 105                 110
   acc agt gct ctg gac gag ctg ctg cag gcg acc cgc gac gcg ggc cgc      503
   Thr Ser Ala Leu Asp Glu Leu Leu Gln Ala Thr Arg Asp Ala Gly Arg
       115                 120                 125
   agg ctg gcg cgt atg gag ggc gcg gag gcg cag cgc cca gag gag gcg      551
   Arg Leu Ala Arg Met Glu Gly Ala Glu Ala Gln Arg Pro Glu Glu Ala
   130                 135                 140                 145
   ggg cgc gcc ctg gcc gcg gtg cta gag gag ctg cgg cag acg cga gcc      599
   Gly Arg Ala Leu Ala Ala Val Leu Glu Glu Leu Arg Gln Thr Arg Ala
                   150                 155                 160
   gac ctg cac gcg gtg cag ggc tgg gct gcc cgg agc tgg ctg ccg gca      647
   Asp Leu His Ala Val Gln Gly Trp Ala Ala Arg Ser Trp Leu Pro Ala
               165                 170                 175
   ggt tgt gaa aca gct att tta ttc cca atg cgt tcc aag aag att ttt      695
   Gly Cys Glu Thr Ala Ile Leu Phe Pro Met Arg Ser Lys Lys Ile Phe
           180                 185                 190
   gga agc gtg cat cca gtg aga cca atg agg ctt gag tct ttt agt gcc      743
   Gly Ser Val His Pro Val Arg Pro Met Arg Leu Glu Ser Phe Ser Ala
       195                 200                 205
   tgc att tgg gtc aaa gcc aca gat gta tta aac aaa acc atc ctg ttt      791
   Cys Ile Trp Val Lys Ala Thr Asp Val Leu Asn Lys Thr Ile Leu Phe
   210                 215                 220                 225
   tcc tat ggc aca aag agg aat cca tat gaa atc cag ctg tat ctc agc      839
   Ser Tyr Gly Thr Lys Arg Asn Pro Tyr Glu Ile Gln Leu Tyr Leu Ser
                   230                 235                 240
   tac caa tcc ata gtg ttt gtg gtg ggt gga gag gag aac aaa ctg gtt      887
   Tyr Gln Ser Ile Val Phe Val Val Gly Gly Glu Glu Asn Lys Leu Val
               245                 250                 255
   gct gaa gcc atg gtt tcc ctg gga agg tgg acc cac ctg tgc ggc acc      935
   Ala Glu Ala Met Val Ser Leu Gly Arg Trp Thr His Leu Cys Gly Thr
           260                 265                 270
   tgg aat tca gag gaa ggg ctc aca tcc ttg tgg gta aat ggt gaa ctg      983
   Trp Asn Ser Glu Glu Gly Leu Thr Ser Leu Trp Val Asn Gly Glu Leu
       275                 280                 285
   gcg gct acc act gtt gag atg gcc aca ggt cac att gtt cct gag gga     1031
   Ala Ala Thr Thr Val Glu Met Ala Thr Gly His Ile Val Pro Glu Gly
   290                 295                 300                 305
   gga atc ctg cag att ggc caa gaa aag aat ggc tgc tgt gtg ggt ggt     1079
   Gly Ile Leu Gln Ile Gly Gln Glu Lys Asn Gly Cys Cys Val Gly Gly
                   310                 315                 320
   ggc ttt gat gaa aca tta gcc ttc tct ggg aga ctc aca ggc ttc aat     1127
   Gly Phe Asp Glu Thr Leu Ala Phe Ser Gly Arg Leu Thr Gly Phe Asn
               325                 330                 335
   atc tgg gat agt gtt ctt agc aat gaa gag ata aga gag acc gga gga     1175
   Ile Trp Asp Ser Val Leu Ser Asn Glu Glu Ile Arg Glu Thr Gly Gly
           340                 345                 350
   gca gag tct tgt cac atc cgg ggg aat att gtt ggg tgg gga gtc aca      1223
   Ala Glu Ser Cys His Ile Arg Gly Asn Ile Val Gly Trp Gly Val Thr
       355                 360                 365
   gag atc cag cca cat gga gga gct cag tat gtt tca taa atgttgtgaa       1272
   Glu Ile Gln Pro His Gly Gly Ala Gln Tyr Val Ser
   370                 375                 380
   actccacttg aagccaaaga aagaaactca cacttaaaac acatgccagt tgggaaggtc   1332
   tgaaaactca gtgcataata ggaacacttg agactaatga aagagagagt tgagaccaat   1392
   ctttatttgt actggccaaa tactgaataa acagttgaag gaaagacatt ggaaaaagct   1452
   tttgaggata atgttactag actttatgcc atggtgcttt cagtttaatg ctgtgtctct   1512
   gtcagataaa ctctcaaata attaaaaagg actgtattgt tgaacagagg gacaattgtt   1572
   ttacttttct ttggttaatt ttgttttggc cagagatgaa ttttacattg gaagaataac   1632
   aaaataagat ttgttgtcca ttgttcattg ttattggtat gtaccttatt acaaaaaaaa   1692
   tgatgaaaac atatttatac tacaaggtga cttaacaact ataaatgtag tttatgtgtt   1752
   ataatcgaat gtcacgtttt tgagaagata gtcatataag ttatattgca aaagggattt   1812
   gtattaattt aagactattt ttgtaaagct ctactgtaaa taaaatattt tataaaacta   1872
   aaaaaaaaaa aaaaaaaaaa aaaaaaaaaa aaaaaa                              1908
<210> 2
   <211> 381
   <212> PRT
   <213> Artificial
<220>
   <223> Synthetic Construct
<400> 2
   Met His Leu Leu Ala Ile Leu Phe Cys Ala Leu Trp Ser Ala Val Leu
   1               5                   10                  15
   Ala Glu Asn Ser Asp Asp Tyr Asp Leu Met Tyr Val Asn Leu Asp Asn
               20                  25                  30
   Glu Ile Asp Asn Gly Leu His Pro Thr Glu Asp Pro Thr Pro Cys Asp
           35                  40                  45
   Cys Gly Gln Glu His Ser Glu Trp Asp Lys Leu Phe Ile Met Leu Glu
       50                  55                  60
   Asn Ser Gln Met Arg Glu Arg Met Leu Leu Gln Ala Thr Asp Asp Val
   65                  70                  75                  80
   Leu Arg Gly Glu Leu Gln Arg Leu Arg Glu Glu Leu Gly Arg Leu Ala
                   85                  90                  95
   Glu Ser Leu Ala Arg Pro Cys Ala Pro Gly Ala Pro Ala Glu Ala Arg
               100                 105                 110
   Leu Thr Ser Ala Leu Asp Glu Leu Leu Gln Ala Thr Arg Asp Ala Gly
           115                 120                 125
   Arg Arg Leu Ala Arg Met Glu Gly Ala Glu Ala Gln Arg Pro Glu Glu
       130                 135                 140
   Ala Gly Arg Ala Leu Ala Ala Val Leu Glu Glu Leu Arg Gln Thr Arg
   145                 150                 155                 160
   Ala Asp Leu His Ala Val Gln Gly Trp Ala Ala Arg Ser Trp Leu Pro
                   165                 170                 175
   Ala Gly Cys Glu Thr Ala Ile Leu Phe Pro Met Arg Ser Lys Lys Ile
               180                 185                 190
   Phe Gly Ser Val His Pro Val Arg Pro Met Arg Leu Glu Ser Phe Ser
           195                 200                 205
   Ala Cys Ile Trp Val Lys Ala Thr Asp Val Leu Asn Lys Thr Ile Leu
       210                 215                 220
   Phe Ser Tyr Gly Thr Lys Arg Asn Pro Tyr Glu Ile Gln Leu Tyr Leu
   225                 230                 235                 240
   Ser Tyr Gln Ser Ile Val Phe Val Val Gly Gly Glu Glu Asn Lys Leu
                   245                 250                 255
   Val Ala Glu Ala Met Val Ser Leu Gly Arg Trp Thr His Leu Cys Gly
               260                 265                 270
   Thr Trp Asn Ser Glu Glu Gly Leu Thr Ser Leu Trp Val Asn Gly Glu
           275                 280                 285
   Leu Ala Ala Thr Thr Val Glu Met Ala Thr Gly His Ile Val Pro Glu
       290                 295                 300
   Gly Gly Ile Leu Gln Ile Gly Gln Glu Lys Asn Gly Cys Cys Val Gly
   305                 310                 315                 320
   Gly Gly Phe Asp Glu Thr Leu Ala Phe Ser Gly Arg Leu Thr Gly Phe
                   325                 330                 335
   Asn Ile Trp Asp Ser Val Leu Ser Asn Glu Glu Ile Arg Glu Thr Gly
                   340                 345                 350
   Gly Ala Glu Ser Cys His Ile Arg Gly Asn Ile Val Gly Trp Gly Val
               355                 360                 365
   Thr Glu Ile Gln Pro His Gly Gly Ala Gln Tyr Val Ser
           370                 375                 380
<210> 3
   <211> 161
   <212> PRT
   <213> Artificial
<220>
   <223> Histone H3 fragment
<400> 3
   Glu Asn Ser Asp Asp Tyr Asp Leu Met Tyr Val Asn Leu Asp Asn Glu
   1               5                   10                  15
   Ile Asp Asn Gly Leu His Pro Thr Glu Asp Pro Thr Pro Cys Asp Cys
               20                  25                  30
   Gly Gln Glu His Ser Glu Trp Asp Lys Leu Phe Ile Met Leu Glu Asn
           35                  40                  45
   Ser Gln Met Arg Glu Arg Met Leu Leu Gln Ala Thr Asp Asp Val Leu
       50                  55                  60
   Arg Gly Glu Leu Gln Arg Leu Arg Glu Glu Leu Gly Arg Leu Ala Glu
   65                  70                  75                  80
   Ser Leu Ala Arg Pro Cys Ala Pro Gly Ala Pro Ala Glu Ala Arg Leu
                   85                  90                  95
   Thr Ser Ala Leu Asp Glu Leu Leu Gln Ala Thr Arg Asp Ala Gly Arg
               100                 105                 110
   Arg Leu Ala Arg Met Glu Gly Ala Glu Ala Gln Arg Pro Glu Glu Ala
           115                 120                 125
   Gly Arg Ala Leu Ala Ala Val Leu Glu Glu Leu Arg Gln Thr Arg Ala
       130                 135                 140
   Asp Leu His Ala Val Gln Gly Trp Ala Ala Arg Ser Trp Leu Pro Ala
   145                 150                 155                 160
   Gly
<210> 4
   <211> 20
   <212> PRT
   <213> Artificial
<220>
   <223> Histone H3 fragment
<400> 4
   Met Ala Arg Thr Lys Gln Thr Ala Arg Lys Ser Thr Gly Gly Lys Ala
   1               5                   10                  15
   Pro Arg Lys Gln
               20
<210> 5
   <211> 20
   <212> PRT
   <213> Artificial
<220>
   <223> Histone H3 fragment
<400> 5
   Ser Thr Gly Gly Lys Ala Pro Arg Lys Gln Leu Ala Thr Lys Ala Ala
   1               5                   10                  15
   Arg Lys Ser Ala
               20
<210> 6
   <211> 20
   <212> PRT
   <213> Artificial
<220>
   <223> Histone H3 fragment
<400> 6
   Leu Ala Thr Lys Ala Ala Arg Lys Ser Ala Pro Ala Thr Gly Gly Val
   1               5                   10                  15
   Lys Lys Pro His
               20
<210> 7
   <211> 20
   <212> PRT
   <213> Artificial
<220>
   <223> Histone H3 fragment
<400> 7
   Pro Ala Thr Gly Gly Val Lys Lys Pro His Arg Tyr Arg Pro Gly Thr
   1               5                   10                  15
   Val Ala Leu Arg
               20
<210> 8
   <211> 20
   <212> PRT
   <213> Artificial
<220>
   <223> Histone H3 fragment
<400> 8
   Arg Tyr Arg Pro Gly Thr Val Ala Leu Arg Glu Ile Arg Arg Tyr Gln
   1               5                   10                  15
   Lys Ser Thr Glu
               20
<210> 9
   <211> 20
   <212> PRT
   <213> Artificial
<220>
   <223> Histone H3 fragment
<400> 9
   Glu Ile Arg Arg Tyr Gln Lys Ser Thr Glu Leu Leu Ile Arg Lys Leu
   1               5                   10                  15
   Pro Phe Gln Arg
               20
<210> 10
   <211> 20
   <212> PRT
   <213> Artificial
<220>
   <223> Histone H3 fragment
<400> 10
   Leu Leu Ile Arg Lys Leu Pro Phe Gln Arg Leu Val Arg Glu Ile Ala
   1               5                   10                  15
   Gln Asp Phe Lys
               20
<210> 11
   <211> 20
   <212> PRT
   <213> Artificial
<220>
   <223> Histone H3 fragment
<400> 11
   Leu Val Arg Glu Ile Ala Gln Asp Phe Lys Thr Asp Leu Arg Phe Gln
   1               5                   10                  15
   Ser Ser Ala Val
               20
<210> 12
   <211> 20
   <212> PRT
   <213> Artificial
<220>
   <223> Histone H3 fragment
<400> 12
   Thr Asp Leu Arg Phe Gln Ser Ser Ala Val Met Ala Leu Gln Glu Ala
   1               5                   10                  15
   Cys Glu Ala Tyr
               20
<210> 13
   <211> 20
   <212> PRT
   <213> Artificial
<220>
   <223> Histone H3 fragment
<400> 13
   Met Ala Leu Gln Glu Ala Cys Glu Ala Tyr Leu Val Gly Leu Phe Glu
   1               5                   10                  15
   Asp Thr Asn Leu
               20
<210> 14
   <211> 20
   <212> PRT
   <213> Artificial
<220>
   <223> Histone H3 fragment
<400> 14
   Leu Val Gly Leu Phe Glu Asp Thr Asn Leu Cys Ala Ile His Ala Lys
   1               5                   10                  15
   Arg Val Thr Ile
               20
<210> 15
   <211> 20
   <212> PRT
   <213> Artificial
<220>
   <223> Histone H3 fragment
<400> 15
   Cys Ala Ile His Ala Lys Arg Val Thr Ile Met Pro Lys Asp Ile Gln
   1               5                   10                  15
   Leu Ala Arg Arg
               20
<210> 16
   <211> 16
   <212> PRT
   <213> Artificial
<220>
   <223> Histone H3 fragment
<400> 16
   Met Pro Lys Asp Ile Gln Leu Ala Arg Arg Ile Arg Gly Glu Arg Ala
   1               5                   10                  15
<210> 17
   <211> 20
   <212> PRT
   <213> Artificial
<220>
   <223> Histone H4 fragment
<400> 17
   Met Ser Gly Arg Gly Lys Gly Gly Lys Gly Leu Gly Lys Gly Gly Ala
   1               5                   10                  15
   Lys Arg His Arg
               20
<210> 18
   <211> 20
   <212> PRT
   <213> Artificial
<220>
   <223> Histone H4 fragment
<400> 18
   Leu Gly Lys Gly Gly Ala Lys Arg His Arg Lys Val Leu Arg Asp Asn
   1 5 10 15
   Ile Gln Gly Ile
   20
<210> 19
   <211> 20
   <212> PRT
   <213> Artificial
<220>
   <223> Histone H4 fragment
<400> 19
   Lys Val Leu Arg Asp Asn Ile Gln Gly Ile Thr Lys Pro Ala Ile Arg
   1 5 10 15
   Arg Leu Ala Arg
   20
<210> 20
   <211> 20
   <212> PRT
   <213> Artificial
<220>
   <223> Histone H4 fragment
<400> 20
   Thr Lys Pro Ala Ile Arg Arg Leu Ala Arg Arg Gly Gly Val Lys Arg
   1 5 10 15
   Ile Ser Gly Leu
   20
<210> 21
   <211> 20
   <212> PRT
   <213> Artificial
<220>
   <223> Histone H4 fragment
<400> 21
   Arg Gly Gly Val Lys Arg Ile Ser Gly Leu Ile Tyr Glu Glu Thr Arg
   1               5                   10                  15
   Gly Val Leu Lys
               20
<210> 22
   <211> 20
   <212> PRT
   <213> Artificial
<220>
   <223> Histone H4 fragment
<400> 22
   Ile Tyr Glu Glu Thr Arg Gly Val Leu Lys Val Phe Leu Glu Asn Val
   1               5                   10                  15
   Ile Arg Asp Ala
               20
<210> 23
   <211> 20
   <212> PRT
   <213> Artificial
<220>
   <223> Histone H4 fragment
<400> 23
   Val Phe Leu Glu Asn Val Ile Arg Asp Ala Val Thr Tyr Thr Glu His
   1               5                   10                  15
   Ala Lys Arg Lys
               20
<210> 24
   <211> 20
   <212> PRT
   <213> Artificial
<220>
   <223> Histone H4 fragment
<400> 24
   Val Thr Tyr Thr Glu His Ala Lys Arg Lys Thr Val Thr Ala Met Asp
   1               5                   10                  15
   Val Val Tyr Ala
               20
<210> 25
   <211> 20
   <212> PRT
   <213> Artificial
<220>
   <223> Histone H4 fragment
<400> 25
   Thr Val Thr Ala Met Asp Val Val Tyr Ala Leu Lys Arg Gln Gly Arg
   1               5                   10                  15
   Thr Leu Tyr Gly
               20
<210> 26
   <211> 13
   <212> PRT
   <213> Artificial
<220>
   <223> Histone H4 fragment
<400> 26
   Leu Lys Arg Gln Gly Arg Thr Leu Tyr Gly Phe Gly Gly
   1               5                   10
<210> 27
   <211> 136
   <212> PRT
   <213> Homo sapiens
<400> 27
   Met Ala Arg Thr Lys Gln Thr Ala Arg Lys Ser Thr Gly Gly Lys Ala
   1               5                   10                  15
   Pro Arg Lys Gln Leu Ala Thr Lys Ala Ala Arg Lys Ser Ala Pro Ala
               20                  25                  30
   Thr Gly Gly Val Lys Lys Pro His Arg Tyr Arg Pro Gly Thr Val Ala
           35                  40                  45
   Leu Arg Glu Ile Arg Arg Tyr Gln Lys Ser Thr Glu Leu Leu Ile Arg
       50                  55                  60
   Lys Leu Pro Phe Gln Arg Leu Val Arg Glu Ile Ala Gln Asp Phe Lys
   65                  70                  75                  80
   Thr Asp Leu Arg Phe Gln Ser Ser Ala Val Met Ala Leu Gln Glu Ala
                   85                  90                  95
   Cys Glu Ala Tyr Leu Val Gly Leu Phe Glu Asp Thr Asn Leu Cys Ala
               100                 105                 110
   Ile His Ala Lys Arg Val Thr Ile Met Pro Lys Asp Ile Gln Leu Ala
           115                 120                 125
   Arg Arg Ile Arg Gly Glu Arg Ala
       130                 135
<210> 28
   <211> 103
   <212> PRT
   <213> Homo sapiens
<400> 28

## Description

### Technical Field

The present invention relates to a therapeutic or prophylactic agent for systemic inflammatory response syndrome (SIRS). Also, the present invention relates to a reagent for quantification of histone and a method of quantification of histone. Furthermore, the present invention relates to a polypeptide complex containing a particular polypeptide and a production method thereof.

### Background Art

Systemic Inflammatory Response Syndrome (SIRS) is defined to be a condition showing at least two items of (1) not more than 36.0°C or not less than 38.0°C, (2) respiratory rate of not less than 20 times/min or PaCO₂<32 mmHg, (3) pulse of not less than 90/min, and (4) leukocytes of not less than 12000/mm³ or less than 4000/mm³, and is a severe pathology. SIRS is divided into SIRS caused by infection, or sepsis, and non-infectious SIRS, based on the etiology. The pathology of SIRS is developed when inflammation is evoked by pathogen associated molecular patterns (PAMPs) derived from an invading microorganism, or damage associated molecular patterns (DAMPs) which are intracellularly-derived substances released from a dead cell and the like. No curative treatment has ever existed heretofore except the administration of an antibiotic against infection, and symptomatic therapy such as infusion and the like has been the only treatment. Particularly, since a treatment method of DAMPs does not exist, a treatment method of DAMPs is desired to be established to improve the survival rate.

Pentraxin 3 (PTX3) is a pattern recognition molecule belonging to the pentraxin family. Pentraxin family is a generic term for proteins having a common pentraxin domain on the C-terminal side, and is classified into short pentraxin and long pentraxin by the characteristics of the primary structure (non-patent document 1). C-reactive protein (CRP), serum amyloid P component (SAP) and the like belong to short pentraxin, and PTX3 belongs to long pentraxin. The primary structure of PTX3 is constituted of N-terminal domain (18 - 178 amino acids) longer than the short pentraxin and pentraxin domain (179 - 381 amino acids) on the C-terminal side, and its higher order structure forms an octamer via a disulfide bond (non-patent document 2).

PTX3 is expressed in rich variety of cell type in response to inflammatory signals and, different from CRP and SAP produced in the liver, characteristically shows topical expression patterns. As a characteristic production mechanism of PTX3, PTX3 stored in the neutrophil granules is extracellularly released when stimulated by bacteria and Toll-like receptor (TLR) agonists. Released PTX3 functions as a constituent protein of a bacterium-capturing and killing structure, which consists of DNA called Neutrophil extracellular traps (NETs) and antibacterial proteins (non-patent document 3). The function of PTX3 in the living body is wide-ranging and, for example, inflammation control, innate immune response, pregnancy maintenance and the like have been reported (non-patent document 4). PTX3 also has a function to bind to many proteins, and exhibits specific functions in coordination with the bound proteins.

The blood concentration of PTX3 has been reported to increase in various infections (non-patent document 4). Particularly in sepsis, it is known that PTX3 concentration, which is normally 2 ng/mL or less, increases to about 200 - 800 ng/mL, and correlates with the survival rate (non-patent document 5). There is also a report stating that PTX3 transgenic mouse is resistant to lethality associated with sepsis (non-patent document 6). From the foregoing, it is assumed that PTX3 with increased blood concentration plays some defensive role against sepsis, though the molecular mechanism thereof has not been elucidated sufficiently.
Garlanda et al., Annu. Rev. Immunol. (2005) 23: 337-336 discloses that PTX3 ineracts with histones.
Daigo et al., Mol. Cell. Proteomics (2012) 11: 1-12 discloses the isolation of PTX3 complexes from patient fluids.

### [Document List] [non-patent documents]

non-patent document 1: Bottazzi, B., et al. (2010) Annual Review of Immunology 28, 157-183
non-patent document 2: Inforzato, A., et al. (2008) J Biol Chem 283, 10147-10161
non-patent document 3: Jaillon, S., et al. (2007) J Exp Med 204, 793-804
non-patent document 4: Mantovani, A., et al. (2008) J Clin Immunol 28, 1-13
non-patent document 5: Mauri, T., et al. (2010) Intensive Care Med 36, 621-629
non-patent document 6: Dias, A. A., et al. (2001) J Leukoc Biol 69, 928-936

### SUMMARY OF THE INVENTION

### Problems to be Solved by the Invention

The present invention aims to provide a useful tool for the treatment or prophylaxis of SIRS. In addition, the present invention aims to provide a useful tool for the diagnosis of SIRS.

### Means of Solving the Problems

The present inventors have conducted intensive studies in an attempt to solve the aforementioned problems and found that pentraxin 3 forms an aggregate with histone, which is one of the DAMPs causing SIRS, and elucidated that the N-terminal domain of pentraxin 3 is involved in the aggregation. The present inventors have conducted further studies and found that administration of a protein relating to the N-terminal domain of pentraxin 3 improves the fatality rate of mice administered with LPS, and verified that the protein is useful for the treatment or prophylaxis of SIRS. Based on these findings, the present inventors conducted further studies and completed the present invention.

Accordingly, the present invention relates to the following.
[1] A polypeptide comprising (ii) a continuous partial sequence of the amino acid sequence shown in SEQ ID NO: 3 having a length of not less than 8 amino acids; or (iii) an amino acid sequence having not less than 70% identity with the amino acid sequence of (ii); which is capable of binding to histone to form a polypeptide aggregate, or a pharmacologically acceptable salt thereof, for use for treating or preventing systemic inflammatory response syndrome, wherein the continuous partial sequence of (ii) comprises any of the following regions:
   (1) a region consisting of the 1st - 50th amino acids of the amino acid sequence shown in SEQ ID NO: 3,
   (2) a region consisting of the 38th - 87th amino acids of the amino acid sequence shown in SEQ ID NO: 3,
   (3) a region consisting of the 75th - 124th amino acids of the amino acid sequence shown in SEQ ID NO: 3, and
   (4) a region consisting of the 112th - 161st amino acids of the amino acid sequence shown in SEQ ID NO: 3, and
   wherein the length of the polypeptide is not more than 100 amino acids.
[2] Use of a polypeptide comprising (ii) a continuous partial sequence of the amino acid sequence shown in SEQ ID NO: 3 having a length of not less than 8 amino acids; or (iii) an amino acid sequence having not less than 70% identity with the amino acid sequence of (ii); which is capable of binding to histone to form a polypeptide aggregate, or a pharmacologically acceptable salt thereof, for quantifying histone in vitro, wherein the continuous partial sequence of (ii) comprises any of the following regions:
   (1) a region consisting of the 1st - 50th amino acids of the amino acid sequence shown in SEQ ID NO: 3,
   (2) a region consisting of the 38th - 87th amino acids of the amino acid sequence shown in SEQ ID NO: 3,
   (3) a region consisting of the 75th - 124th amino acids of the amino acid sequence shown in SEQ ID NO: 3, and
   (4) a region consisting of the 112th - 161st amino acids of the amino acid sequence shown in SEQ ID NO: 3, and
   wherein the length of the polypeptide is not more than 100 amino acids.
[3] The use according to [2], wherein the polypeptide or a pharmacologically acceptable salt thereof is immobilized on a solid phase carrier.
[4] A method of quantifying histone, comprising (1) a step of contacting a polypeptide comprising (ii) a continuous partial sequence of the amino acid sequence shown in SEQ ID NO: 3 having a length of not less than 8 amino acids; or (iii) an amino acid sequence having not less than 70% identity with the amino acid sequence of (ii); which is capable of binding to histone to form a polypeptide aggregate, or a pharmacologically acceptable salt thereof with a histone-containing sample to form a polypeptide aggregate comprising the polypeptide or a pharmacologically acceptable salt thereof and histone, and (2) a step of quantifying the polypeptide aggregate obtained in step (1), wherein the continuous partial sequence of (ii) comprises any of the following regions:
   (1) a region consisting of the 1st - 50th amino acids of the amino acid sequence shown in SEQ ID NO: 3,
   (2) a region consisting of the 38th - 87th amino acids of the amino acid sequence shown in SEQ ID NO: 3,
   (3) a region consisting of the 75th - 124th amino acids of the amino acid sequence shown in SEQ ID NO: 3, and
   (4) a region consisting of the 112th - 161st amino acids of the amino acid sequence shown in SEQ ID NO: 3, and
   wherein the length of the polypeptide is not more than 100 amino acids.
[5] The method according to [4], wherein the histone contained in the polypeptide aggregate is quantified in step (2) by an immunological method by using an antibody that specifically recognizes histone.
[6] A polypeptide complex comprising (1) a polypeptide comprising (ii) a continuous partial sequence of the amino acid sequence shown in SEQ ID NO: 3 having a length of not less than 8 amino acids; or (iii) an amino acid sequence having not less than 70% identity with the amino acid sequence of (ii); which is capable of binding to histone to form a polypeptide aggregate, or a pharmacologically acceptable salt thereof, and (2) histone or a pentraxin 3 N-terminal domain-binding fragment thereof, wherein the continuous partial sequence of (ii) comprises any of the following regions:
   (1) a region consisting of the 1st - 50th amino acids of the amino acid sequence shown in SEQ ID NO: 3,
   (2) a region consisting of the 38th - 87th amino acids of the amino acid sequence shown in SEQ ID NO: 3,
   (3) a region consisting of the 75th - 124th amino acids of the amino acid sequence shown in SEQ ID NO: 3, and
   (4) a region consisting of the 112th - 161st amino acids of the amino acid sequence shown in SEQ ID NO: 3, and
   wherein the length of the polypeptide is not more than 100 amino acids.
[7] The polypeptide complex according to [6], which is a polypeptide aggregate.
[8] A method of producing a polypeptide complex comprising (1) a polypeptide comprising (ii) a continuous partial sequence of the amino acid sequence shown in SEQ ID NO: 3 having a length of not less than 8 amino acids; or (iii) an amino acid sequence having not less than 70% identity with the amino acid sequence of (ii); which is capable of binding to histone to form a polypeptide aggregate, or a pharmacologically acceptable salt thereof, and (2) histone or a pentraxin 3 N-terminal domain-binding fragment thereof, comprising a step of contacting the polypeptide or a pharmacologically acceptable salt thereof of (1) with histone or a pentraxin 3 N-terminal domain-binding fragment thereof of (2),
   wherein the continuous partial sequence of (ii) comprises any of the following regions:
   (1) a region consisting of the 1st - 50th amino acids of the amino acid sequence shown in SEQ ID NO: 3,
   (2) a region consisting of the 38th - 87th amino acids of the amino acid sequence shown in SEQ ID NO: 3,
   (3) a region consisting of the 75th - 124th amino acids of the amino acid sequence shown in SEQ ID NO: 3, and
   (4) a region consisting of the 112th - 161st amino acids of the amino acid sequence shown in SEQ ID NO: 3, and
   wherein the length of the polypeptide is not more than 100 amino acids.
[9] The production method according to [8], wherein the polypeptide complex is a polypeptide aggregate.

According to the present invention, the treatment or prophylaxis of SIRS caused by histone can be performed by administering the polypeptide as mentioned above to SIRS patients to induce formation of a polypeptide aggregate containing the polypeptide and histone, thereby neutralizing the cytotoxic activity of histone on, for example, vascular endothelial cells. According to the present invention, moreover, in an in vitro method, extracellular histone present in, for
example, blood can be detected or quantified by mixing a sample such as blood and the like collected from a subject and the mentioned above and detecting the formed polypeptide aggregate containing the polypeptide and histone, whereby SIRS caused by histone can be diagnosed.

### Brief Description of the Drawings

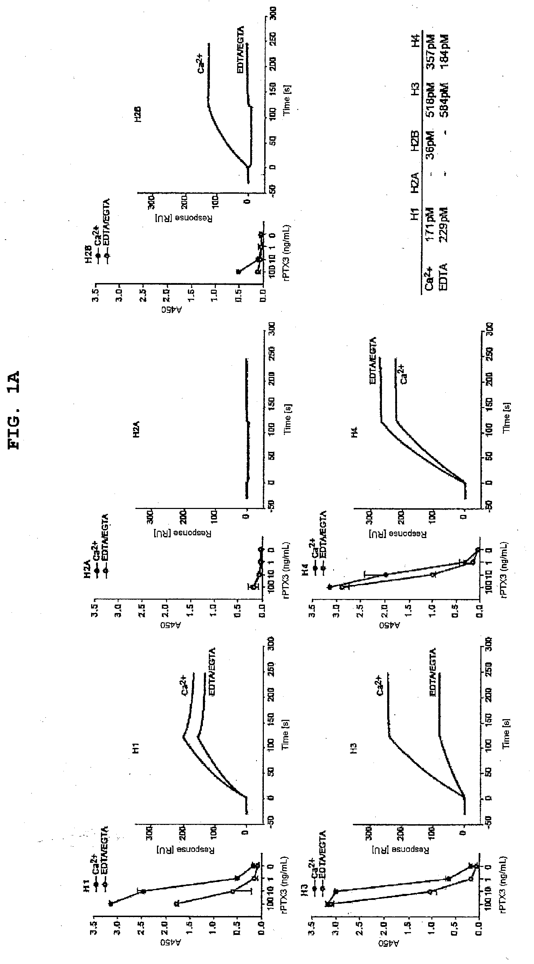

Fig. 1A shows the measurement results of the property of binding of tag-free full-length PTX3 and each histone variant by binding assay (left) and BIAcore (right). In the binding assay, HRP-conjugated anti-PTX3 antibody (PPZ1228) was used for the detection. In BIAcore, the reactivity with 1 nM tag-free full-length PTX3 was examined. Histones specifically used were histone H1, histone H2A, histone H2B, histone H3 and histone H4. The measurement values of the binding with each histone variant was calculated from the information of sensorgram wherein tag-free full-length PTX3 was serially diluted by 1/2 in 5 stages from 1 nM and reacted (Table).
Fig. 1B shows UV-visible absorption spectrum of PTX3-histone aggregate. 0.1 mg/mL human histone H4 was mixed with equal volume of various concentrations of Myc-tagged full-length PTX3, and UV-visible absorption spectrum was measured. A graph of absorbance at 310 nm is shown at the top.
Fig. 2A shows expression and purification of PTX3 domain proteins. The upper Figure shows illustration of Myc- and His-tagged PTX3 domain proteins, and the lower Figure shows SYPRO Ruby staining results of Myc- and His-tagged PTX3 domain proteins (Reducing: under reducing condition, Non-reducing: under non-reducing condition).
Fig. 2B shows binding property of PTX3 domain proteins and each histone variant. The results of the binding assay of PTX3 domain proteins expressed by animal cells and each histone variant are shown. The detection was performed using HRP-conjugated anti-Myc antibody.

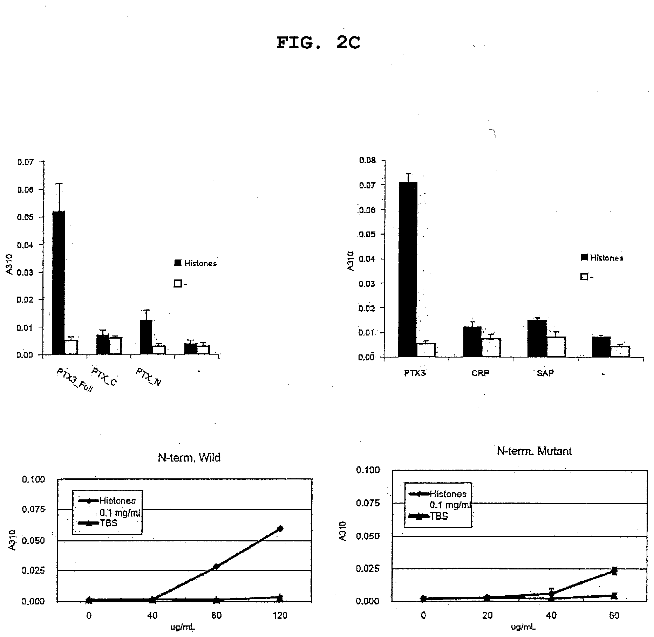

Fig. 2C shows the capacity of aggregation of PTX3 domain proteins, CRP or SAP and histone. The upper left figure shows the measurement results of absorbance at 310 nm of a mixture of 0.5 µM PTX3 domain proteins and 1.0 mg/mL Calf Thymus Histones at 1:1 vol. The upper right figure shows the measurement results of absorbance at 310 nm of a mixture of 0.5 mg/mL each pentraxin and 1.0 mg/mL Calf Thymus Histones at 1:1 vol. The lower left figure and the lower right figure show the measurement results of absorbance at 310 nm of a mixture of a wild-type or oligomer formation capacity-deficient mutant of PTX3 N-terminal domain having each concentration and 0.1 mg/mL Calf Thymus Histones at 1:1 vol.
Fig. 3A shows the results confirming an influence of pentraxin family on the toxic activity of extracellular histone on HUVEC. After culturing for 1 hr in a medium concurrently containing 100 µg/mL Calf Thymus Histones and APC (200 nM), PTX3 (40 µg/mL), CRP (40 µg/mL) or SAP (40 µg/mL), the level of toxic activity was measured by FACS.
Fig. 3B shows the results confirming an influence of PTX3 on the histone decomposition by APC. 25 µg/mL of each histone variant was mixed with 100 nM APC and 25 µg/mL PTX3, and a sample incubated at 37°C for 1 hr was stained with SYPRO Ruby.
Fig. 3C shows the results confirming an influence of PTX3 N-terminal domain on extracellular histone on HUVEC. The left figure shows the measurement results by FACS of toxic activity after culture for 1 hr in a medium concurrently containing 20 µg/mL human histone H4 and 100, 200 or 400 µg/mL N-terminal domain. The right figure shows the results confirming PTX3 concentration-dependent peak shift.
Fig. 3D shows the results confirming an influence of PTX3 N-terminal domain on the mortality of LPS-administered mouse. The left figure shows a death curve and the right figure shows the measurement results of inflammation markers.
Fig. 4 shows the measurement results of binding levels between immobilized each recombinant histone and tag-free recombinant human PTX3 (rhPTX3) by the binding assay (ELISA). HRP-conjugated anti-PTX3 monoclonal antibody (PPZ-1228) was used for the detection. Assay was performed with duplicate wells for each point, and means and standard deviations were calculated from three independent experiments.
Fig. 5A shows a suppressive effect of Myc-tagged human PTX3 fragments on histone-mediated cytotoxicity.
Fig. 5B shows the effect of pentraxins other than PTX3 on histone-mediated cytotoxicity.
Fig. 5C shows survival rates of mice administered intraperitoneally with LPS, and administered with or without PTX3 N-terminal domain fragment.
Fig. 5D shows survival rates of mice subjected to cecal ligation and puncture (CLP), and administered with or without PTX3 N-terminal domain fragment.
Fig. 5E shows time-course changes of plasma IL-6 and VEGF levels measured by ELISA in LPS-administrated mice treated with or without PTX3. Bar graphs represent means and standard error (±SEM).
Fig. 6A shows aggregation activity of PTX3 and other pentraxins with histone. Concentrations indicated in the graph are concentrations after mixture.
Fig. 6B shows a schematic method of the determination of aggregation stoichiometry.
Fig. 6C shows Histone-PTX3 aggregation stoichiometry. Each mixing molar ratios was indicated above the band.
Fig. 6D shows the results of CD spectrum measurements of histone H4-PTX3 complex.
Fig. 7A schematically shows Histone H3 and H4 fragments.
Fig. 7B shows cytotoxic activity of histone H3 and H4 fragments. Representative results from three independent experiments are respectively shown.
Fig. 7C shows cytotoxic activity values of histone H3 and H4 fragments.
Fig. 7D shows a suppressive effect of PTX3 on cytotoxic activity of H3-6, H3-8, H3-11 and H4-9 fragments. Representative results from three independent experiments are shown.
Fig. 8A shows binding activity of each histone variant with PTX3 or fragment thereof as measured by the binding assay.
Fig. 8B shows binding activity of each histone variant with PTX3 N-terminal fragments (wild-type or mutant) as measured by the binding assay.
Fig. 9A shows the results of surface plasmon resonance (SPR) measurement of the binding of each histone variant and PTX3 or a fragment thereof.
Fig. 9B shows the results of surface plasmon resonance (SPR) measurement of the binding of each histone variant and PTX3 N-terminal fragments (wild type or mutant).
Fig. 9C is a table summarizing affinities calculated from Fig. 9A and Fig. 9B.
Fig. 10A shows a suppressive effect of PTX3 on cytotoxicity of each histone variant.
Fig. 10B shows a suppressive effect of PTX3 N-terminal fragment on cytotoxicity of each histone variant.
Fig. 11A shows the results observing dose-dependent light scattering of histone-PTX3 aggregation. The concentrations indicated in the graph are those after mixing.
Fig. 11B shows the results of aggregation of each histone variant and PTX3 fragment.
Fig. 11C shows dose-dependent precipitate formation of histone-PTX3 aggregation.
Fig. 11D shows aggregation stoichiometry between histone and PTX3 N-terminal domain. Each mixing molar ratios is indicated above the band.
Fig. 11E shows the results of concentration setting of histone and PTX3 used for CD spectrum measurement.
Fig. 12A shows PTX3-binding activity of histone H3 and H4.
Fig. 12B shows a suppressive effect of PTX3 N-terminal domain on cytotoxicity of histone H3 and H4 fragments.
Fig. 12C shows the results of CD spectrum measurements of histone H3 and H4 fragments-PTX3 complex.
Fig. 13 shows a schematic figure of designed various fragments of human PTX3 N-terminal domain.
Fig. 14 shows protocols for expression, lysis and purification of human PTX3 N-terminal domain fragments.
Fig. 15 shows the qualification results of various fragments of human PTX3 N-terminal domain.
Fig. 16 shows the evaluation results of histone-binding activity of various fragments of human PTX3 N-terminal domain.
Fig. 17 shows the evaluation results of aggregate-formation capacity of various fragments of human PTX3 N-terminal domain with histone.
Fig. 18 shows an expressed amount and yield of various fragments of human PTX3 N-terminal domain in 500 ml culture scale.
Fig. 19 shows a suppressive effect of various fragments of human PTX3 N-terminal domain on cytotoxic activity of histone.

### Description of Embodiments

The present invention provides a therapeutic or prophylactic agent for systemic inflammatory response syndrome (SIRS), which contains, as an active ingredient, the polypeptide mentioned above capable of binding to histone to form a polypeptide aggregate (hereinafter sometimes to be referred to as "the polypeptide of the present invention") or a pharmacologically acceptable salt thereof.

PTX3 is a known protein belonging to the protein family generally called pentraxin family, and is a protein belonging to long pentraxin. PTX3 is generally derived from vertebrata.

Examples of the vertebrata include mammal, birds, fish, amphibian, reptile and the like. While the mammal is not particularly limited, examples thereof include experiment animals such as rodents (e.g., mouse, rat, hamster, guinea pig and the like), rabbit and the like; domestic animals such as swine, bovine, goat, horse, sheep, mink and the like; companion animals such as dog, cat and the like; and primates such as human, monkey, Macaca mulatta, marmoset, orangutan, chimpanzee and the like. Examples of the birds include chicken, quail, duck, goose, turkey, ostrich, emu, camel bird, guinea fowl, pigeon and the like. The vertebrata is preferably mammal, more preferably human.

In the present specification, when polypeptide and polynucleotide are "derived from living organism X", it means that the amino acid sequence of the polypeptide or the nucleic acid sequence of the polynucleotide is the same as the amino acid sequence of the polypeptide or the nucleic acid sequence of the polynucleotide, which are naturally expressed in the living organism X.

PTX3 derived from human typically consists of a single strand polypeptide having a full-length 381 amino acids. A representative amino acid sequence of PTX3 polypeptide derived from human is registered as Genebank Accession No. AAH39733 (SEQ ID NO: 2). Also, a representative base sequence encoding PTX3 polypeptide derived from human is registered as Genebank Accession No. BC039733 (SEQ ID NO: 1).

Generally, when PTX3 polypeptide expressed in a cell is extracellularly secreted, its N-terminal signal peptide is cleaved during the process to be a mature PTX3 polypeptide. In the present specification, the PTX3 polypeptide is preferably a mature PTX3 polypeptide. For example, the 1st - 17th amino acids from the N-terminal of the amino acid sequence of the PTX3 derived from human is a signal peptide that is cleaved in the process of extracellular secretion to be a mature polypeptide. Therefore, mature PTX3 polypeptide derived from human representatively contains the 18th - 381st amino acid sequence of the amino acid sequence shown in SEQ ID NO: 2.

The N-terminal domain of PTX3 is a region on the N-terminal side of the pentraxin domain of the aforementioned PTX3 polypeptide (preferably mature PTX3 polypeptide) or a part thereof. The pentraxin domain is a domain common to the members of the pentraxin superfamily such as CRP (C-reactive Protein), SAP (Serum amyloid P component) and the like, and is registered in the NCBI Conserved Domain as Accession No. cd00152. Therefore, those of ordinary skill in the art can identify the pentraxin domain based on any PTX3 sequence information, and identify the N-terminal domain of the PTX3. The pentraxin domain of PTX3 derived from human representatively corresponds to a region consisting of the 179th - 380th amino acids of the amino acid sequence shown in SEQ ID NO: 2. Therefore, the N-terminal domain of PTX3 derived from human is generally a region consisting of the 1st - 178th amino acids of the amino acid sequence shown in SEQ ID NO: 2 or a part thereof, preferably, a region consisting of the 18th - 178th amino acids of the amino acid sequence shown in SEQ ID NO: 2 or a part thereof. When PTX3 is derived from human, the N-terminal domain thereof is preferably the 18th - 178th amino acid from the N-terminal. The amino acid sequence of a region consisting of the 18th - 178th amino acids of the amino acid sequence shown in SEQ ID NO: 2 is shown in SEQ ID NO: 3.

When the N-terminal domain of PTX3 is a part of a region on the N-terminal side of the pentraxin domain of PTX3 polypeptide, its length for having an activity to bind to histone to form a polypeptide aggregate is at least 8 amino acids, for example, not less than 10 amino acids, preferably not less than 30 amino acids, more preferably not less than 50 amino acids, further preferably not less than 100 amino acids, still more preferably not less than 150 amino acids (e.g., 151, 152, 153, 154, 155, 156, 157, 158, 159, 160 amino acid).

In a further aspect, N-terminal domain of the PTX3 derived from human contains any of the following regions:
(1) a region consisting of the 18th - 67th amino acids of the amino acid sequence shown in SEQ ID NO: 2 (a region consisting of the 1st - 50th amino acids of the amino acid sequence shown in SEQ ID NO: 3),
(2) a region consisting of the 55th - 104th amino acids of the amino acid sequence shown in SEQ ID NO: 2 (a region consisting of the 38th - 87th amino acids of the amino acid sequence shown in SEQ ID NO: 3),
(3) a region consisting of the 92nd - 141st amino acids of the amino acid sequence shown in SEQ ID NO: 2 (a region consisting of the 75th - 124th amino acids of the amino acid sequence shown in SEQ ID NO: 3), and
(4) a region consisting of the 129th - 178th amino acids of the amino acid sequence shown in SEQ ID NO: 2 (a region consisting of the 112th - 161st amino acids of the amino acid sequence shown in SEQ ID NO: 3).

As shown in the below-mentioned Examples, each region of (1) - (4) has an activity to bind to histone to form a polypeptide aggregate.

In one embodiment of the invention the polypeptide contains an amino acid sequence the same or substantially the same as the N-terminal domain of PTX3, in particular the amino acid sequence substantially the same as the amino acid sequence of the N-having not less than 70%, preferably not less than 80%, furthermore preferably not less than 90%, particularly preferably not less than 95%, most preferably not less than 99% identity with the amino acid sequence of the N-terminal domain of PTX3, in particular, the continuous partial sequence of the amino acid sequence shown in SEQ ID No: 3 having a length of not less than 8 amino acids. As used herein, the "identity" means a ratio (%) of the same overlapping amino acids relative to the total amino acid residue at the optimal alignment when two amino acid sequences are aliened using a mathematical algorithm known in the pertinent technical field.

Examples of the amino acid sequence substantially the same as the amino acid sequence of the N-terminal domain of PTX3 include (1) an amino acid sequence wherein one or more (preferably 1 - 30, preferably about 1 - 10, more preferably 1 or 2) amino acids are deleted in the amino acid sequence of the N-terminal domain of PTX3, (2) an amino acid sequence wherein one or more (preferably about 1 - 30, preferably 1 - 10, more preferably 1 or 2) amino acids are added in the amino acid sequence of the N-terminal domain of PTX3, (3) an amino acid sequence wherein one or more (preferably 1 - 30, preferably about 1 - 10, more preferably 1 or 2) amino acids are inserted in the amino acid sequence of the N-terminal domain of PTX3, (4) an amino acid sequence wherein one or more (preferably 1 - 30, preferably about 1 - 10, more preferably 1 or 2) amino acids are substituted by other amino acids in the amino acid sequence of the N-terminal domain of PTX3, or (5) a combination of such amino acid sequences and the like.

When an amino acid sequence is inserted, deleted, added or substituted as mentioned above, the position of the insertion, deletion, addition or substitution is not particularly limited as long as the polypeptide containing such amino acid sequence has an activity to bind to histone to form a polypeptide aggregate. Examples of the amino acid sequence substantially the same as the amino acid sequence of the N-terminal domain of PTX3, which is usable in the present invention include the amino acid sequence of its homologue in the vertebrata other than human mentioned above and the like.

To maintain the activity to bind to histone to form a polypeptide aggregate even when the amino acid sequence contains insertion, deletion, addition or substitution, the 47th, 49th and 103rd cysteine residues of the amino acid sequence shown in SEQ ID NO: 2 are preferably preserved.

As the polypeptide containing an amino acid sequence substantially the same as the amino acid sequence of the N-terminal domain of PTX3, a polypeptide containing the aforementioned amino acid sequence substantially the same as the amino acid sequence of the N-terminal domain of PTX3 and having the activity with nature substantially equivalent to that of the polypeptide containing the amino acid sequence of the N-terminal domain of PTX3 is preferable.

The example of the activity with nature substantially equivalent include an activity to bind to histone to form a polypeptide aggregate. As used herein, the "substantially equivalent nature" means that the properties thereof are qualitatively (e.g., physiologically or pharmacologically) equivalent. Therefore, the activity of a polypeptide consisting of the above-mentioned substantially the same amino acid sequence is preferably equivalent. However, quantitative elements such as the level of activity (e.g., about 0.01 - about 100-fold, preferably about 0.1 - about 10-fold, more preferably 0.5 - 2-fold), the molecular weight of polypeptide and the like may be different.

The length of the polypeptide of the present invention having an activity to bind to histone to form a polypeptide aggregate is not more than 100 amino acids, preferably not more than amino acids, from the aspects of easy preparation and stability of polypeptide.

Examples of the polypeptide of the present invention include a polypeptide consisting of an amino acid sequence the same or substantially the same as the amino acid sequence of the N-terminal domain of PTX3 (e.g., SEQ ID NO: 3) (e.g., the N-terminal domain of PTX3 alone). The polypeptide of the present invention is not PTX3 full-length polypeptide (including mature form and immature form).

The N-terminal domain of PTX3 and the polypeptide of the present invention have an activity to bind to histone to form a polypeptide aggregate. Here, the "form an aggregate" in the present specification means that the N-terminal domain of PTX3 and histone are bound by a specific interaction to form a water-insoluble dense assembled state. In the present specification, the "polypeptide aggregate" means a water-insoluble mass assemblage containing the N-terminal domain of PTX3 and histone.

Histone is one kind of a protein constituting chromatin (chromosome) of eucaryotes, and has an activity to bind to DNA. In the present specification, histone is generally derived from vertebrata, preferably derived from a mammal, most preferably derived from human. Histone encompasses H1, H2A, H2B, H3 and H4. The N-terminal domain of PTX3 and the polypeptide of the present invention generally have an activity to bind to at least one kind of histone selected from the group consisting of H1, H2A, H2B, H3 and H4, preferably at least one kind of histone selected from the group consisting of H1, H3 and H4, more preferably each of H1, H3 and H4, to form a polypeptide aggregate.

The presence or absence of an activity to bind to histone to form a polypeptide aggregate can be confirmed by, for example, visual observation of formation of a polypeptide aggregate. When, for example, equal volumes of 1.0 mg/ml histone solution (in buffer (150 mM NaCl, 20 mM HEPES, 4 mM CaCl₂, 0.005% surfactant P20 (pH 7.4))) and 1.0 mg/ml evaluation target polypeptide solution (in the aforementioned buffer) are mixed, and the presence of a particulate substance can be confirmed by visual observation, the evaluation target polypeptide can be judged to have an activity to bind to histone to form a polypeptide aggregate. The formation of a polypeptide aggregate can be more clearly confirmed by using an electron microscope in the visual observation.

The presence or absence of an activity to bind to histone to form a polypeptide aggregate can also be confirmed by measuring the UV-visible absorption spectrum. When, for example, equal volumes of 0.1 mg/ml histone solution (in buffer (150 mM NaCl, 20 mM HEPES, 4 mM CaCl₂, 0.005% surfactant P20 (pH 7.4))) and various concentrations of evaluation target polypeptide solution (in the aforementioned buffer) are mixed, the spectrum is measured and a dose-dependent increase in the absorption spectrum of UV-visible light (e.g., 310 nm) due to the scattering of aggregates is observed, or an increase in the absorption spectrum compared to the same concentration of histone alone, the N-terminal domain of PTX3 alone, or the polypeptide of the present invention alone is observed, the evaluation target polypeptide can be judged to have an activity to bind to histone to form a polypeptide aggregate.

Furthermore, the presence or absence of an activity to bind to histone to form a polypeptide aggregate can also be confirmed by immunochromatograpy, Ouchterlony method or immunity nephelometry.

When formation of a polypeptide aggregate could be confirmed in at least one method among the above-mentioned methods, an evaluation target polypeptide is judged to have an activity to bind to histone to form a polypeptide aggregate.

The polypeptide contained in the therapeutic or prophylactic agent of the present invention contains an amino acid sequence being the same as the aforementioned amino acid sequence of the N-terminal domain of PTX3, and it may be a polypeptide consisting of the aforementioned amino acid sequence (e.g., N-terminal domain of PTX3 alone) but not a PTX3 full-length protein (including mature form and immature form). The amino acid length of the polypeptide is not more than 100 amino acids, more preferably not more than 50 amino acids.

In the present specification, a polynucleotide specified by the amino acid sequence has, according to of the conventional manner of peptide indication, N-terminal (amino terminal) on the left side and C-terminal (carboxyl terminal) on the right side. The C-terminal of the polypeptide of the present invention may be any of carboxyl group (-COOH), carboxylate (-COO-), amide (-CONH₂) and ester (-COOR). In addition, when a polypeptide containing the N-terminal domain of PTX3 of the present invention has a carboxyl group (or carboxylate) at a site other than the C-terminal, the carboxyl group may be amidated or esterified in the present invention. Furthermore, the above-mentioned polypeptide also includes composite polypeptides such as a polypeptide wherein the amino group of the N-terminal amino acid residue (e.g., methionine residue) is protected by a protecting group, a polypeptide wherein the N-terminal glutamine residue produced by cleavage in vivo is converted to pyroglutamic acid, a polypeptide wherein a substituent on the side chain of intramolecular amino acid is protected by a suitable protecting group, and a glycoprotein wherein a sugar chain is bonded and the like, and the like.

The polypeptide of the present invention may be a free form or a pharmacologically acceptable salt. As such salt, a salt with a pharmacologically acceptable acid, a base and the like is used. As such salt, for example, a salt with inorganic acid (e.g., hydrochloric acid, phosphoric acid, hydrobromic acid, sulfuric acid), a salt with organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), an alkali metal salt (e.g., sodium salt, potassium salt), an alkaline earth metal salt (e.g., calcium salt, barium salt), a magnesium salt, an aluminum salt and the like are used.

A drug that potentiates the function of the polypeptide of the present invention, a complex wherein other protein is added to the polypeptide of the present invention, and a polypeptide wherein a plurality of the polypeptide of the present invention is linked are also usable as the active ingredient of the therapeutic or prophylactic agent of the present invention.

The polypeptide of the present invention is preferably isolated. The "isolated" means that an operation to remove factors other than the object component has been applied to be deviated from the naturally-present state. The purity of the "isolated polypeptide X" (percentage of polypeptide X in the total weight of polypeptide) is generally not less than 70%, preferably not less than 80%, more preferably not less than 90%, most preferably substantially 100%.

The polypeptide of the present invention can be produced from the cell or tissue of the aforementioned vertebrata by a protein purification method known per se. To be specific, a tissue or cell of the animal is homogenized, extracted with acid and the like, and the extract is purified and isolated by a combination of chromatographys such as reversed-phase chromatography, ion exchange chromatography, affinity-chromatography and the like.

In addition, the polypeptide of the present invention can also be produced by culturing, by a method known per se, a transformant introduced with an expression vector containing a polynucleotide containing a nucleotide sequence encoding the polypeptide or a complementary sequence thereof, and separating the polypeptide from the obtained culture.

The above-mentioned polynucleotide containing a nucleotide sequence encoding the polypeptide or a complementary sequence thereof may be a DNA or an RNA, or a DNA/RNA chimera, with preference given to DNA. In addition, the nucleic acid may be double stranded or single stranded. When it is a double stranded, it may be a double stranded DNA, a double stranded RNA or a DNA:RNA hybrid.

Examples of the DNA containing a nucleotide sequence encoding the above-mentioned polypeptide or a complementary sequence thereof include chromosome DNA, cDNA derived from a cell of the aforementioned vertebrata that expresses the above-mentioned polypeptide, any tissue or organ containing the cell, synthetic DNA and the like. The chromosome DNA and cDNA encoding the above-mentioned polypeptide can be directly amplified by Polymerase Chain Reaction (PCR method) or Reverse Transcriptase-PCR (RT-PCR method) and using chromosome DNA fraction and total RNA or mRNA fraction prepared from the aforementioned cell or tissue each as a template. Alternatively, chromosome DNA and cDNA encoding the polypeptide of the present invention can be each cloned from a known chromosome DNA library and cDNA library prepared by inserting a fragment of chromosome DNA, cDNA and total RNA or mRNA, which is prepared from the above-mentioned cell or tissue into a suitable vector, by a colony or plaque hybridization method or PCR method and the like.

The production of the above-mentioned polypeptide by utilizing a transformant can be specifically performed according to the method of the below-mentioned Examples.

Furthermore, the polypeptide of the present invention can also be produced according to a known peptide synthesis method. The peptide synthesis method may be any of a solid phase synthesis process and a liquid phase synthesis process. A partial peptide or amino acid capable of constituting the polypeptide of the present invention and the remaining portion are condensed, a protecting group is removed when the resultant product has a protecting group, and thereby the object polypeptide can be produced. Condensation and removal of the protecting group can be performed according to a method known per se.

In addition, the polypeptide of the present invention can also be produced by cleaving a PTX3 protein with a suitable peptidase.

The polypeptide obtained as mentioned above can be purified by a method known per se. Examples of the purification method include solvent extraction, distillation, column chromatography, liquid chromatography, recrystallization, and a combination of these and the like. When the polypeptide obtained by the above-mentioned method is a free form, the free form can be converted into a suitable salt (preferably, pharmacologically acceptable salt) by a known method or a method analogous thereto, and when the polypeptide is obtained as a salt, the salt can be converted into a free form or other salt (preferably, pharmacologically acceptable salt) by a known method or a method analogous thereto.

The polypeptide of the present invention or a pharmacologically acceptable salt thereof can be mixed with a pharmacologically acceptable carrier as necessary to give a pharmaceutical composition, and can be used as a therapeutic or prophylactic agent for SIRS. SIRS in a mammal (preferably human) can be prevented or treated by administering a prophylactically or therapeutically effective amount of the polypeptide of the present invention or a pharmacologically acceptable salt thereof to the mammal. While the mammal to be the administration subject is preferably human, it may also be a mammal other than human. Examples of such mammal include mouse, rat, rabbit, dog, cat, horse, sheep, bovine, goat, swine, miniature swine, hairless swine, monkey and the like.

In the present specification, "SIRS" means a generic term of diseases belonging to systemic inflammatory response syndrome. In the present invention, SIRS is preferably a disease relating to damage associated molecular patterns (DAMPs). In the present specification, the diseases relating to DAMPs mean the same as the diseases caused by DAMPs. Examples of the DAMPs that cause SIRS include histone (e.g., histone H1, histone H2A, histone H2B, histone H3, histone H4 etc.), HMGB1 (High Mobility Group Box 1), S100 protein, heat shock protein, hyaluronic acid decomposition product, ATP, urine acid, heparin sulfate, DNA and the like. In the present invention, DAMPs are preferably histones from among the above, and more preferably histone H1, H3 or H4. SIRS can be divided into, for example, infectious disease (infectious SIRS) and non-infectious disease (non-infectious SIRS). Infectious SIRS among SIRS means a disease caused by infection with pathogen such as bacterium, fungi, virus and the like. Specific examples of the diseases in infectious SIRS include bacterium infection, fungi infection, virus infection, parasite infection and the like. Non-infectious SIRS among SIRS means a disease caused by an intracellular component released by cell death and the like. Specific examples of the diseases in non-infectious SIRS include pancreatitis, burn, trauma, ischemic reperfusion disorder, surgical stress, rhabdomyolysis and the like. These diseases are related to histone (particularly histone H1, H3 or H4) at a certain ratio. In the present invention, SIRS is preferably non-infectious SIRS.

In the present invention, as the pharmacologically acceptable carrier used for a pharmaceutical composition, various organic or inorganic carrier substances conventionally used as preparation materials are used. Examples thereof include excipient, lubricant, binder and disintegrant for solid preparations; and solvent, solubilizing agent, suspending agent, isotonicity agent, buffering agent, soothing agent for liquid preparations and the like. In addition, where necessary, preparation additives such as preservative, antioxidant, colorant, sweetening agent and the like can also be used. As these carriers, compounds known per se, which are usable for pharmaceutical composition, can be used, and commercially available products can be preferably utilized. In addition, the amount of various carriers can be appropriately determined by those of ordinary skill in the art.

Examples of the dosage form of the above-mentioned pharmaceutical composition include oral preparations such as tablet, capsule (including soft capsule and microcapsule), granule, powder, syrup, emulsion, suspension and the like; and parenteral preparations such as injection (e.g., subcutaneous injection, intravenous injection, intramuscular injection, intraperitoneal injection etc.), external preparation (e.g., preparations for nasal administration, dermal preparation, ointment etc.), suppository (e.g., rectal suppository, vaginal suppository etc.), pellet, drip infusion, sustained-release preparation (e.g., sustained-release microcapsule etc.) and the like. Pharmaceutical compositions such as these can be produced by a method conventionally used in the preparation technical field, for example, the method described in the Japanese Pharmacopoeia and the like.

The dose of the therapeutic or prophylactic agent for SIRS of the present invention is preferably an amount sufficient for the polypeptide of the present invention or a pharmacologically acceptable salt thereof to bind to histone to form an aggregate and neutralize histone in the body of a treatment target mammal (e.g., in blood). The dose of the therapeutic or prophylactic agent of the present invention in parenteral administration varies depending on the subject of administration, target organ, symptom, administration method and the like. For example, it is about 10 - 1000 mg, preferably about 100 - 500 mg, more preferably about 200 - 400 mg, as the weight of the polypeptide of the present invention, for patients with body weight 60 kg. Even when the subject of administration is other than human, an amount based on the body weight 60 kg can be administered.

The present invention also provides a reagent for quantification of histone, containing, the above-mentioned polypeptide of the present invention or a pharmacologically acceptable salt thereof. The contents (definition and embodiment etc.) of each term relating to the reagent for quantification of the present invention are the same as those described for the above-mentioned therapeutic or prophylactic agent of the present invention.

The reagent for quantification of histone of the present invention is based on the finding that the above-mentioned polypeptide of the present invention or a pharmacologically acceptable salt thereof and histone form an aggregate. Therefore, the reagent for quantification of the present invention is not particularly limited as long as it is in an embodiment permitting measurement and quantification of such aggregate. For example, the reagent for quantification of the present invention may consist only of the above-mentioned polypeptide of the present invention or a pharmacologically acceptable salt thereof, provided as a solid and prepared when in use with a suitable buffer and the like. Alternatively, it may be dissolved in advance at a suitable concentration in the aforementioned buffer and the like, and provided as a liquid product.

In a further embodiment, the reagent for quantification of the present invention may be provided as the above-mentioned polypeptide of the present invention or a pharmacologically acceptable salt thereof immobilized on a solid phase carrier. Examples of the carrier include, but are not limited to, plastic plate (e.g., 96 well plate), magnetic bead, latex bead, membrane and the like.

When the reagent for quantification of the present invention is used, histone can be quantified by performing (1) a step of contacting a polypeptide comprising an amino acid sequence the same or substantially the same as the amino acid sequence of the N-terminal domain of pentraxin 3 capable of binding to histone to form a polypeptide aggregate, or a pharmacologically acceptable salt thereof with a histone-containing sample to form a polypeptide aggregate comprising the polypeptide or a pharmacologically acceptable salt thereof and histone, and (2) a step of quantifying the polypeptide aggregate obtained in step (1). As the quantification method of histone, a method of confirming the presence or absence of the activity to bind to histone to form a polypeptide aggregate explained above and the like can be utilized.

Specific examples of the quantification method of histone include measurement of UV-visible absorption spectrum. For example, equal volumes of various known concentrations (e.g., 0.01, 0.1, 1, 10 mg/ml) of histone solution (in buffer (150 mM NaCl, 20 mM HEPES, 4 mM CaCl₂, 0.005% surfactant P20 (pH 7.4))) and a given concentration (e.g., 1 mg/ml) of a solution of the polypeptide of the present invention or a pharmacologically acceptable salt thereof (in the aforementioned buffer) are mixed, the absorption spectrum at a given wavelength (e.g., 310 nm) is measured and a standard curve is prepared. The absorption spectrum when a histone solution at an unknown concentration is used thereon is measured and the concentration of histone can be determined using the aforementioned standard curve.

Other method for quantifying the aforementioned histone (or polypeptide aggregate) includes a method of detecting/quantifying histone contained in a polypeptide aggregate by an immunological method using an antibody that specifically recognizes histone. For example, when a solid phase carrier immobilizing the aforementioned polypeptide of the present invention or a pharmacologically acceptable salt thereof is contacted with a histone-containing sample, histone is captured by the polypeptide of the present invention or a pharmacologically acceptable salt thereof, and a polypeptide aggregate containing the polypeptide of the present invention or a pharmacologically acceptable salt thereof and histone can be formed on the solid phase carrier. The polypeptide aggregate is contacted with an antibody that specifically recognizes histone, and histone contained in the polypeptide aggregate is detected/quantified by an immunological method using an antigen specific binding of the antibody. Examples of the immunological method include, but are not limited to, enzyme immunoassay (EIA method), radioimmunoassay (RIA method), fluorescent immunoassay (FIA method), luminescence immunoassay, latex agglutination, Western blot method, immunochromatograpy method and the like.

In the present specification, Examples of the antibody include, but are not limited to, natural antibodies such as polyclonal antibody, monoclonal antibody (mAb) and the like, chimera antibody that can be produced using a gene recombinant technique, humanized antibody, single strand antibody, human antibody that can be produced using human antibody-producing transgenic animal and the like, antibody fragment produced by Fab expression library, and binding fragments (F(ab')₂, Fab', Fab, Fv, sFv, dsFv, dAb etc.) thereof. Preferred antibody is polyclonal antibody, monoclonal antibody or binding fragments thereof.

The "specific recognition" means that affinity of a certain antibody for a particular antigen is higher than the affinity for other antigen.

The production methods of a polyclonal antibody, a monoclonal antibody, and a binding fragment thereof that specifically recognize particular antigen are well known to those of ordinary skill in the art.

An antibody that specifically recognizes histone is preferably an antibody that specifically recognizes histone H1, H2A, H2B, H3 or H4 derived from human, more preferably an antibody that specifically recognizes histone H1, H3 or H4 derived from human. Using, for example, an antibody that specifically recognizes histone H3 derived from human as the antibody, histone H3 derived from human can be specifically quantified.

A method of immobilizing the polypeptide of the present invention or a pharmacologically acceptable salt thereof on a solid phase carrier is known in the pertinent technical field and is not particularly limited. For example, the polypeptide of the present invention or a pharmacologically acceptable salt thereof can be immobilized on a solid phase carrier by contacting a solution of the polypeptide of the present invention or a pharmacologically acceptable salt thereof in an appropriate buffer with the solid phase carrier.

The kind of the buffer is not particularly limited as long as it permits immobilization. Examples thereof include citrate buffer, MES buffer, phosphate buffer, HEPES buffer, Tris-HCl buffer, carbonate buffer, borate buffer and the like. When a plastic plate is adopted as a solid phase carrier, citrate buffer, MES buffer, carbonate buffer and borate buffer are preferably used.

While the pH of the buffer is also not particularly limited as long as it permits immobilization, when a plastic plate is adopted as a solid phase carrier, the pH is preferably weak acidic ranging from 3.5 to 4.0 or weak alkaline ranging from 8.5 to 9.6, to increase the adsorption efficiency of the polypeptide of the present invention or a pharmacologically acceptable salt thereof to the plastic plate.

When a plastic plate is adopted as a solid phase carrier, the buffer to be used for immobilization is preferably citrate buffer or MES buffer at pH 3.5 - 4.0, or carbonate buffer or borate buffer at pH 8.5 - 9.6, most preferably carbonate buffer at pH 8.5 - 9.6.

A solid phase carrier immobilizing the polypeptide of the present invention or a pharmacologically acceptable salt thereof is preferably blocked by an appropriate blocking agent to suppress non-specific adsorption of histone and an antibody that specifically recognizes histone. While the kind of the blocking agent is not particularly limited as long as it suppresses non-specific adsorption, for example, protein such as skim milk, casein, BSA, gelatin, normal serum and the like, Blockmaster CE510, CE210 (manufactured by JSR Life Sciences Corporation), a compound represented by the formula (1) and the like can be mentioned.

H₂N-(C₂H₄NH)ₙ-R¹-(C₂H₄O)ₘ-R² ... (1)

wherein n=2 - 20, m=2 - 200, R¹ is a single bond, a phenylene group or an alkylene group having 1 - 10 carbon atoms, and R² is a hydrogen atom or an alkyl group having 1 - 3 carbon atoms.

Since histone has a strong positive electric charge and non-specifically adsorbs to BSA, CE510, CE210 or a compound represented by the formula (1) is preferably used as a blocking agent.

The concentration of a blocking agent can be appropriately determined by those of ordinary skill in the art to suppress non-specific adsorption. When CE510 or CE210 is used as a blocking agent, the concentration is generally 0.1 - 1.0% (w/v), preferably 0.1 - 0.3% (w/v).

Blocking is performed by treating a solid phase carrier with a solution of a blocking agent generally at 4 - 37°C for about 1 - 12 hr.

A histone-containing sample used for contacting with a solid phase carrier immobilizing the polypeptide of the present invention or a pharmacologically acceptable salt thereof preferably has a pH adjusted to weak alkaline ranging from 8.0 to 9.6 to enhance bindability to the polypeptide of the present invention or a pharmacologically acceptable salt thereof and increase detection sensitivity. The pH can be adjusted by adding an appropriate buffer (Tris-HCl buffer, carbonate buffer etc.).

The histone-containing sample preferably contains NaCl at, for example, 0.45 - 1.2 M, preferably 0.75 - 1.2 M, more preferably 0.75 - 0.9 M, to enhance binding of histone to the polypeptide of the present invention or a pharmacologically acceptable salt thereof and suppress non-specific adsorption of histone.

The reagent for quantification of the present invention can also be processed into a histone quantification kit, which further contains a substance usable for quantification of histone (particularly, quantification of the above-mentioned polypeptide aggregate). Specific examples of such substance include a buffer for diluting the reagent for quantification of the present invention and histone-containing sample, reaction container, positive control (histone-containing sample having a known histone content), negative control, labeling substance (e.g., fluorescence dye, enzyme etc.), and instructions describing histone quantification method and the like. When, for example, histone is quantified by the aforementioned immunological method, the kit can contain a solid phase carrier immobilizing the polypeptide of the present invention or a pharmacologically acceptable salt thereof, an antibody that specifically recognizes histone, a blocking agent (preferably, CE510, CE210 or a compound represented by the formula (1)), a buffer (Tris-HCl buffer, carbonate buffer etc.) at pH 8.0 - 9.6, concentrated NaCl solution (e.g., not less than 1.2M, not less than 1.0 M), a secondary antibody and the like. These factors can also be mixed in advance where necessary. Using such quantification kit, histone can be quantified more conveniently.

In one embodiment, the histone quantification kit of the present invention contains the following constitutions:
(1) a plastic plate immobilizing the polypeptide of the present invention or a pharmacologically acceptable salt thereof, or a solution of the polypeptide of the present invention or a pharmacologically acceptable salt thereof in a buffer at pH 3.5 - 4.0 or 8.5 - 9.6 (e.g., carbonate buffer at pH 8.5 - 9.6) and a plastic plate,
(2) an antibody that specifically recognizes histone,
(3) a blocking agent (preferably, CE510, CE210 or a compound represented by the formula (1)),
(4) a buffer (Tris-HCl buffer, carbonate buffer etc.) at pH 8.0 - 9.6 (for histone-containing sample preparation), and
(5) a concentrated NaCl solution (e.g., not less than 1.2M, not less than 1.0 M) (for histone-containing sample preparation).

Each constitution is as described in the explanation of other method of quantifying the aforementioned polypeptide aggregate.

Using the aforementioned reagent for quantification of histone or quantification method, amount (concentration) of histone in a sample can be examined. Applying this, histone in a sample collected from, for example, a mammal can be detected or quantified to diagnose whether the mammal is affected with SIRS. Therefore, the present invention further provides a diagnostic reagent for SIRS, which contains the above-mentioned polypeptide of the present invention or a pharmacologically acceptable salt thereof. The contents (definition and embodiment etc.) of each term relating to the diagnostic reagent of the present invention are the same as those described for the above-mentioned therapeutic or prophylactic agent, reagent for quantification of histone and quantification method of the present invention.

SIRS to be the diagnosis target for the diagnostic reagent of the present invention is preferable a disease relating to DAMPs, and such DAMPs are preferably histones. Among them, histone H1, H3 and H4 are more preferable. While SIRS includes infectious disease (infectious SIRS) and non-infectious disease (non-infectious SIRS), non-infectious SIRS is preferable. Specific disease corresponding to SIRS and diseases relating to histone (or histone H1, H3 or H4), and examples of DAMPs are as mentioned above.

The diagnostic reagent of the present invention utilizes formation of an aggregate of the polypeptide of the present invention or a pharmacologically acceptable salt thereof and histone, and histone in a sample collected from a diagnosis target can be detected or quantified using the diagnostic reagent of the present invention, and whether the target is affected with SIRS can be determined based on the presence or absence or the amount of histone in the sample. While the diagnosis target in the present invention is preferably human, it may be a mammal other than human. Examples of such mammal include mouse, rat, rabbit, dog, cat, horse, sheep, bovine, goat, swine, miniature swine, hairless swine, monkey and the like. Examples of the sample to be collected from a diagnosis target include blood, plasma, serum, extravascular fluid, interstitial fluid, cerebrospinal fluid, synovial fluid, pleural fluid, lymph fluid, saliva, seminal fluid, tear, urine and the like. Of these, preferred samples in the present invention are blood, plasma, serum, saliva, seminal fluid, tear, and urine, and more preferred samples are blood, plasma and serum, since invasion in the diagnosis target is less.

The diagnostic reagent of the present invention may be the above-mentioned polypeptide of the present invention or a pharmacologically acceptable salt thereof alone, or may further contain a pharmacologically acceptable carrier. Examples of the pharmacologically acceptable carrier when the diagnosis drug of the present invention is prepared as a liquid include various carriers conventionally used as preparation materials, for example, diluent, solvent, solubilizing agents, suspending agent, isotonicity agent, buffering agent and the like. As these carriers, compounds known per se, which are usable for conventional diagnostic reagent, can be used, and commercially available products can be preferably utilized. In addition, the amount of various carriers can be appropriately determined by those of ordinary skill in the art. The diagnostic reagent of the present invention is not limited to liquid alone, and can take any dosage form such as solid preparation, powder preparation and the like, and can be formulated by a method conventionally used in the technical field of various preparations.

The diagnostic reagent of the present invention is contacted with, for example, a sample (e.g., blood) collected from a diagnosis target (e.g., human), an aggregate formed that contains the polypeptide of the present invention and histone is measured, histone (preferably, histone H4) in the sample is detected or quantified, whereby whether the target is affected with SIRS or has a high risk of being affected with SIRS can be judged.

A method of detecting or quantifying histone in a sample by using the diagnostic reagent of the present invention is not particularly limited as long as it can examine the presence of a polypeptide aggregate containing the polypeptide of the present invention or a pharmacologically acceptable salt thereof and histone. For example, a method of confirming the presence or absence of an activity to bind to histone to form a polypeptide aggregate explained above and the like can be used.

In the present invention, while the judgment of whether the target is affected with SIRS or has a high risk of being affected with SIRS is not particularly limited, it can be performed by comparing the detection or quantification results of histone in a sample with the results obtained from a normal target. As a result of the comparison, when histone is present in the sample, or when the amount of histone in the sample has relatively increased, the target can be judged to have affected with SIRS or have a high risk of being affected with SIRS.

In the present invention, when a target affected with SIRS is treated, the judgment for observation of the effect and progress of the treatment is not particularly limited. It can be performed by comparing the detection or quantification results of histone in a sample with the results obtained from a normal target, the results after being affected, and the results during the treatment. As a result of the comparison, when the amount of histone in the sample has relatively decreased, it can be judged that a treatment effect on the target has been obtained, and the treatment was effective.

The diagnostic reagent of the present invention can also be processed into a SIRS diagnosis kit further containing a reagent usable for a detection or quantification method of histone in a sample and the like. Specific examples of such reagent include a buffer for diluting the reagent and the above-mentioned sample, reaction container, positive control (histone-containing sample having a previously-measured histone content), negative control, labeling substance (e.g., fluorescence dye, enzyme etc.), and instructions describing examination protocol and the like. These factors can also be mixed in advance where necessary. Using such diagnosis kit, SIRS can be diagnosed more conveniently.

Also described is a method of judging the presence or absence of affection with SIRS, which comprises (1) a step of contacting a sample collected from a target with the above-mentioned polypeptide of the present invention or a pharmacologically acceptable salt thereof, and (2) a step of detecting or quantifying histone in the sample.

The contents (definition and embodiment etc.) of each term relating to the judgment method are the same as those described for the above-mentioned therapeutic or prophylactic agent for SIRS of the present invention, and the above-mentioned diagnostic reagent for SIRS of the present invention. Particularly, the presence or absence of affection with SIRS can be judged according to the contents described for the above-mentioned diagnostic reagent of the present invention. Using the judgment method not only the presence or absence of affection with SIRS but also whether the risk of being affected with SIRS is high can also be judged according to the contents described for the above-mentioned diagnostic reagent of the present invention.

The embodiments of the diagnostic reagent of the present invention and a method for use thereof are the same as those described above relating to the aforementioned reagent for quantification and the quantification method of histone of the present invention.

The present invention also provides a polypeptide complex containing the above-mentioned polypeptide of the present invention or a pharmacologically acceptable salt thereof, and histone or a PTX3 N-terminal domain binding fragment thereof, and a production method thereof.

The "polypeptide complex" refers to a state wherein at least two kinds of polypeptides are associated, and may be water-soluble or water-insoluble. The polypeptide complex of the present invention is preferably a water-insoluble polypeptide aggregate.

The contents (definition and embodiment etc.) of each term relating to the polypeptide complex of the present invention and the production method thereof are the same as those described above. Histone to be used for the polypeptide complex of the present invention and a production method thereof is generally at least one kind of histone selected from the group consisting of H1, H2A, H2B, H3 and H4, preferably at least one kind of histone selected from the group consisting of H1, H3 and H4.

Histone to be used for the polypeptide complex of the present invention and a production method thereof is generally derived from vertebrata. Examples of the vertebrata include those recited above. The vertebrata is preferably a mammal, more preferably human.

A representative amino acid sequence of human-derived histone H3 polypeptide is registered as Genebank Accession No. AAN10051 (SEQ ID NO: 27).

A representative amino acid sequence of human-derived histone H4 polypeptide is registered as Genebank Accession No. AAM83108 (SEQ ID NO: 28).

The site of the PTX3 N-terminal domain-binding fragment of histone to be used for the polypeptide complex or a production method thereof of the present invention is not particularly limited as long as it binds to (preferably forms an aggregate with) the N-terminal domain of PTX3 (preferably PTX3 derived from human).

When a PTX3 N-terminal domain-binding fragment of human-derived histone H3 is used, the fragment contains
a region consisting of the 10th - 29th amino acids (H3-2, SEQ ID NO: 5),
a region consisting of the 50th - 69th amino acids (H3-6, SEQ ID NO: 9),
a region consisting of the 70th - 89th amino acids (H3-8, SEQ ID NO: 11),
a region consisting of the 80th - 99th amino acids (H3-9, SEQ ID NO: 12),
a region consisting of the 110th - 119th amino acids (H3-11, SEQ ID NO: 14), or
a region consisting of the 110th - 129th amino acids (H3-12, SEQ ID NO: 15)
(preferably H3-6, 8, 9 or 11, more preferably H3-6, 8 or 11) in the amino acid sequence shown in SEQ ID NO: 27.

When a PTX3 N-terminal domain-binding fragment of human-derived histone H4 is used, the fragment contains
a region consisting of the 50th - 69th amino acids (H4-6, SEQ ID NO: 22),
a region consisting of the 60th - 79th amino acids (H4-7, SEQ ID NO: 23),
a region consisting of the 70th - 89th amino acids (H4-8, SEQ ID NO: 24), or
a region consisting of the 80th - 99th amino acids (H4-9, SEQ ID NO: 25)
(preferably H4-6, 8 or 9, more preferably H4-9) of the amino acid sequence shown in SEQ ID NO: 28.

The length of the PTX3 N-terminal domain-binding fragment of histone to be used for the polypeptide complex or a production method thereof of the present invention is not particularly limited as long as it binds to (preferably forms an aggregate with) the N-terminal domain of PTX3 (preferably PTX3 derived from human). In one embodiment, the length of the fragment can be not more than 100 amino acids, not more than 80 amino acids, not more than 60 amino acids, not more than 40 amino acids, not more than 30 amino acids, not more than 25 amino acids, not more than 24 amino acids, not more than 23 amino acids, not more than 22 amino acids, not more than 21 amino acids, or 20 amino acids.

The content of the polypeptide of the present invention contained in the polypeptide complex of the present invention (preferably polypeptide aggregate) is generally 10 - 90 (polypeptide w/w)%, preferably 40 - 80 (polypeptide w/w)%, more preferably 50 - 60 (polypeptide w/w)%.

The content of histone or a PTX3 N-terminal domain-binding fragment thereof in the polypeptide complex of the present invention (preferably polypeptide aggregate) is generally 10 - 90 (polypeptide w/w)%, preferably 40 - 80 (polypeptide w/w)%, more preferably 50 - 60 (polypeptide w/w)%.

Histone or a PTX3 N-terminal domain-binding fragment thereof contained in the polypeptide complex of the present invention (preferably polypeptide aggregate) may be only one kind (H1, H2A, H2B, H3 or H4), or plural kinds (2, 3, 4 or 5 kinds) of histones or PTX3 N-terminal domain-binding fragments thereof.

The polypeptide complex of the present invention (preferably polypeptide aggregate) may
(1) consist of the polypeptide of the present invention and histone or a PTX3 N-terminal domain-binding fragment thereof, or
(2) comprise a factor (polypeptide) other than the polypeptide of the present invention and histone or a PTX3 N-terminal domain-binding fragment thereof.

In the case of (2), the content of the polypeptide of the present invention and histone or a PTX3 N-terminal domain-binding fragment thereof, which are contained in the polypeptide complex of the present invention (preferably polypeptide aggregate), can be appropriately determined according to the use of the polypeptide complex of the present invention.

The polypeptide complex of the present invention (preferably polypeptide aggregate) is preferably isolated. The purity of the "isolated polypeptide complex" (percentage of polypeptide complex in the total weight of polypeptide) is generally not less than 70%, preferably not less than 80%, more preferably not less than 90%, most preferably substantially 100%.

The polypeptide complex of the present invention (preferably polypeptide aggregate) can be produced by contacting the polypeptide of the present invention or a pharmacologically acceptable salt thereof with histone or a PTX3 N-terminal domain-binding fragment thereof.

In the contacting step, for example, the above-mentioned polypeptide of the present invention or a pharmacologically acceptable salt thereof, and histone or a PTX3 N-terminal domain binding fragment thereof may be, without any particularly limitation, each dissolved or suspended in a suitable buffer and then used. Such buffer may be any as long as it does not inhibit the binding and aggregation of the both molecules and, for example, phosphate buffered saline, Tris buffered saline, HEPES buffer and the like can be mentioned. In addition, the pH of the buffer is generally pH 4 - 10, preferably pH 6 - 8.

The above-mentioned polypeptide of the present invention or a pharmacologically acceptable salt thereof, and histone or a PTX3 N-terminal domain-binding fragment thereof can be brought into contact by, for example, mixing solutions in which the both molecules are respectively dissolved or suspended. Alternatively, one of the polypeptide of the present invention, and histone or a PTX3 N-terminal domain-binding fragment thereof is bound onto a solid phase carrier, and a solution containing the other molecule may be contacted therewith. The mixing ratio in this case is not particularly limited as long as a polypeptide complex (preferably polypeptide aggregate) can be formed, and generally, histone or a PTX3 N-terminal domain-binding fragment thereof is 0.25 - 8 mol, preferably 0.5 - 4 mol, more preferably 1 - 2 mol, relative to 1 part by weight (or mol) of the polypeptide of the present invention.

The both molecules may be brought into contact under any conditions in the presence or absence of a divalent ion (preferably calcium ion) as long as the polypeptide complex of the present invention (preferably polypeptide aggregate) can be formed. As shown in the Examples to be mentioned below, since the polypeptide of the present invention and histone are more stably bound in the presence of a divalent ion (preferably calcium ion), the both molecules are brought into contact in the presence of a divalent ion (preferably calcium ion) in a preferable embodiment.

The temperature and time of contact of the both molecules are not particularly limited. The temperature is generally 0 - 50°C, preferably 4 - 40°C, more preferably 25 - 37°C. The time is generally 1 sec - 10 hr, preferably 1 min - 2 hr, more preferably 5 min - 0.5 hr.

The polypeptide complex (preferably polypeptide aggregate) produced as mentioned above can be thereafter collected by using a suitable antibody and the like, and may be further isolated and purified. The methods of collection, isolation and purification are not particularly limited, and methods known per se can be used. For example, when a polypeptide aggregate is isolated, the polypeptide aggregate of the present invention can be obtained by collecting insoluble fractions from the reaction mixture by centrifugation and the like, and removing the supernatant. The production of a polypeptide complex (preferably polypeptide aggregate) can be confirmed by, for example, immunoprecipitation or the above-mentioned method for confirming the formation of a polypeptide aggregate.

The polypeptide complex (preferably polypeptide aggregate) of the present invention is useful as, for example, a research reagent for analyzing the interaction between PTX3 and histone in SIRS, a positive control in the above-mentioned diagnostic reagent and the like.

### Examples

The present invention is explained in more detail in the following by referring to Examples.

### Experimental Example 1. ELISA binding assay

Recombinant human histone (NEB) was adjusted to a concentration of 1 µg/mL in TBS, added to a 96 well ELISA plate, and immobilized at 4°C overnight. The solution was discarded, a blocking buffer (TBS, 0.1% Triton-XlOO, 1% BSA) was added, and the plate was incubated at room temperature for 2 hr. The plate was washed four times with a wash buffer (TBS, 0.1% Triton-XlOO), recombinant PTX3 diluted to various concentrations with the blocking buffer was added to each well, and reacted at room temperature for 1 hr. After washing four times with the wash buffer, HRP-labeled antibody diluted with the blocking buffer was added, and reacted at room temperature for 1 hr. After washing four times with the wash buffer, TMB solution was added to carry out a color development reaction for 30 min, and the absorbance at 450 nm was measured. Assay was performed using all the buffers containing 4 mM CaCl₂ or 4 mM EDTA.

### Experimental Example 2. BIAcore measurement

For the measurement, BIAcore 3000 or T200 (GE Healthcare) was used. Recombinant human histone was immobilized on CM5 sensor chip by an amine coupling kit (GE Healthcare). The reaction with recombinant PTX3 was performed using a buffer (150 mM NaCl, 20 mM HEPES, 0.005% surfactant P20 (pH 7.4)) at a flow rate of 20 µl/min. Measurement was performed using the buffer containing 2 mM CaCl₂ or 3 mM EDTA. The sensor chip was regenerated in 1M sodium acetate (10 µL, pH 7.2) and 10 mM NaOH (10 µL).

### Experimental Example 3. Cloning, expression and purification of recombinant PTX3

Tag-free PTX3 (rPTX3) was expressed and purified according to a previous report (Savchenko, A., et al. (2008) The Journal of Pathology 215, 48-55). Animal cell expression constructs of Myc- and His-tagged partial- and full-length PTX3 were produced by amplifying sequences encoding N-terminal domain human PTX3 (1 - 178 amino acids), C-terminal domain human PTX3 (179 - 381 amino acids) having human PTX3 signal sequence (1 - 17 amino acids) linked to the N-terminal, and full-length human PTX3 (1 - 381 amino acids), and recloning them into the NotI/XbaI site of pEF4/Myc-His B vector (Invitrogen). The expression vectors were transfected by FreeStyle™MAX CHO system (Invitrogen), and stable cells were established using Zeocin™ (Invitrogen). Proteins were purified from the culture supernatant of each stable cell by using HisTrap HP (1 mL, GE Healthcare Life Sciences). Escherichia coli expression constructs of His-tagged N-terminal PTX3 were produced by totally synthesizing sequences encoding N-terminal domain human PTX3 (18 - 178 amino acids) having TEV sequence (ENLYFQG) linked to the N-terminal, and N-terminal domain human PTX3 wherein the 47th, 49th, 103rd cysteine residues were replaced with serine residue, as a sequence optimized for Escherichia coli expression, and recloning them into NdeI/XhoI site of pCold II vector (TAKARA BIO). BL21-CodonPlus-RIL (Agilent)was transformed with the expression vector and the expression was performed by a conventional method. The collected Escherichia coli was lysed with BugBuster (MERCK) and the protein was purified by HisTrap HP (1 mL, GE Healthcare Life Sciences).

### Experimental Example 4. Cytotoxic assay

Normal human umbilical vein endothelial cells (HUVECs) were cultured in EGM2 medium (Clonetics). The cells after culture were washed with PBS, added with Opti-MEM medium containing an product of incubation of activated protein C (APC), PTX3, C reactive protein (CRP) or Serum amyloid P component (SAP) with histone for 1 hr, and the cells were further cultured for 1 hr. Propidium iodide (PI) was added to the medium after culture at a final concentration of 10 µg/mL, and the cells were stained for 10 min. The cells were washed with PBS, detached and analyzed by flow cytometry.

### Experimental Example 5. Mouse experiment

Escherichia coli-expressed His-tagged N-terminal PTX3 protein (18 - 178 amino acids, 5 mg/kg) was intraperitoneally administered to male C57BL/6 mice, LPS (16 mg/kg) was intraperitoneally administered 2 hr later, and the survival was observed for 8 days. At 0, 6 and 24 hr from the LPS administration, heparin plasma was collected, and the concentrations of mouse IL-6 and mouse VEGF were measured using ELISA kit (R&D).

For cecal ligation and puncture (CLP) treatment, male C57BL/6 mouse was anesthetized, laparotomy was performed, and cecum was exposed and ligated. A hole is made with an injection needle at the middle between the ligated site and cecum peripheral site, and the abdomen is closed. Four hours from the CLP treatment, the N-terminal domain of PTX3 (5 mg/kg) and gentamicin (5 mg/kg), or gentamicin (5 mg/kg) alone were (was) administered to the mouse.

### <Results>

### 1. Aggregation reaction by PTX3-histone binding

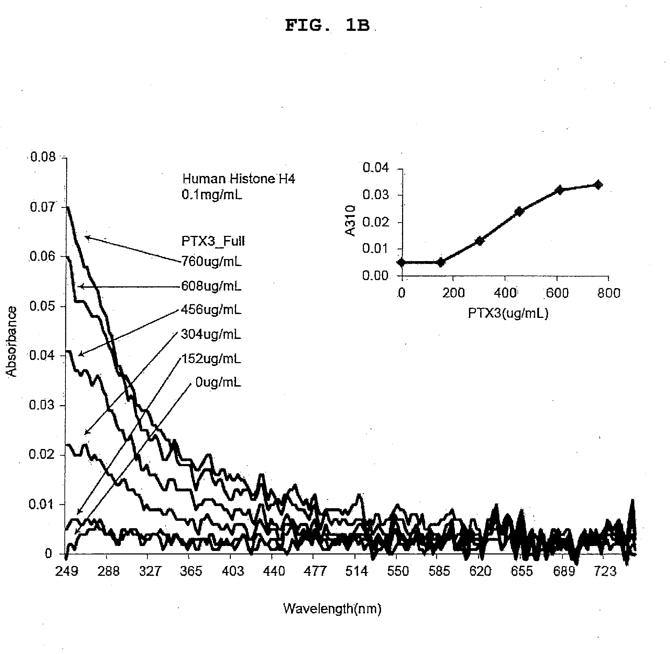

The binding property of each histone variant and PTX3 was analyzed by binding assay and SPR measurement with BIAcore. As a result, each histone variant showed different binding property (Fig. 1A). Particularly histone H1, H3 and H4 showed strong binding affinity for PTX3. Surprisingly, the affinity obtained by SPR measurement was extremely high. The binding signal and dissociation signal were carefully observed, and the reaction was found to be closer to non-specific reaction rather than equilibrium reaction (Fig. 1A). From the above-mentioned results, it was assumed that the binding of PTX3 and histone was based on a mechanism different from a general equilibrium reaction. In addition, an aggregate-like substance was visually confirmed in a mixture of PTX3 and histone, and a dose-dependent spectrum was found in the measurement of UV-visible absorption spectrum (Fig. 1B). Since an increase in the UV-visible absorption spectrum is not found in general protein binding, it was clarified that an aggregation reaction took place between PTX3 and histone, and the spectrum shown in Fig. 1B was considered to have been caused by the scattering of the aggregates including the both.

### 2. Determination of important PTX3 domain for PTX3-histone aggregation reaction

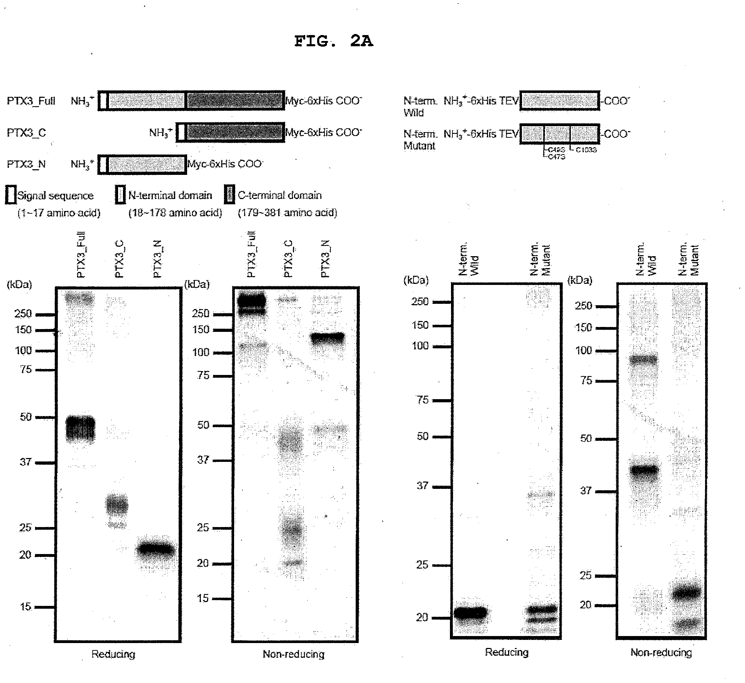

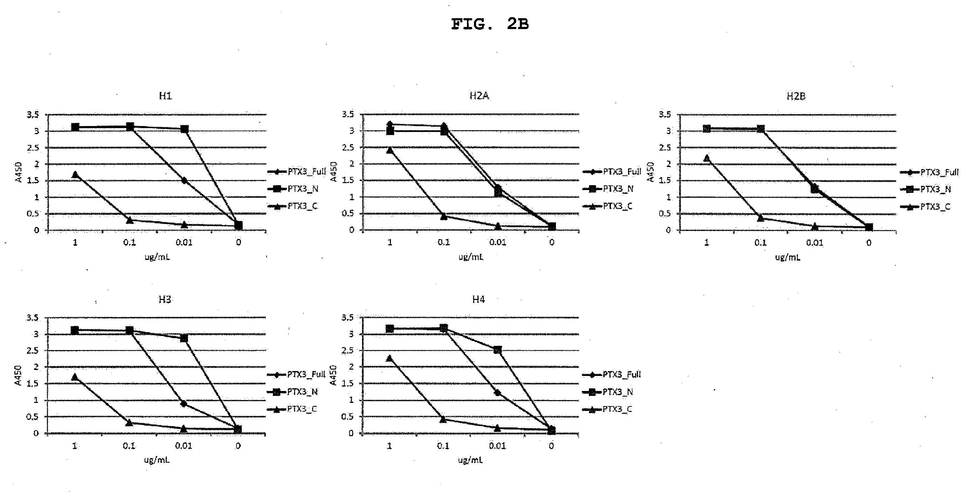

Then, to confirm which domain of PTX3 is involved in the aggregation with histone, a binding assay was performed using PTX3 domain recombinant proteins and N-terminal oligomer formation mutants (Fig. 2A). As a result, a strong bindability to N-terminal domain was observed in histone H1, H3 and H4, (Fig. 2B). Moreover, although weak, bindability to C-terminal domain was also observed (Fig. 2B). Therefore, the aggregation reaction with histone was confirmed to also find an aggregation reaction in the N-terminal domain (Fig. 2C). In addition, the N-terminal domain that lost the oligomer formation capacity by replacing the 47th, 49th, 103rd cysteine residues with serine residue showed a decrease in both the binding capacity and aggregation reaction (Fig. 2B, C). From these results, it is considered that the 47th - 103rd amino acid region of PTX3 is important for the aggregation reaction with histone. To further confirm that the aggregation reaction of PTX3 and histone occurs in the PTX3 N-terminal domain specific to long pentraxin, the aggregation capacity of CRP and SAP, which have been reported to have a short N-terminal domain and binding capacity to histone, with histone was confirmed to find no aggregation capacity (Fig. 2C).

### 3. Suppression of vascular endothelial cytotoxic activity of histone by PTX3 and improvement of rate of death caused by LPS administration in PTX3-administrered mouse

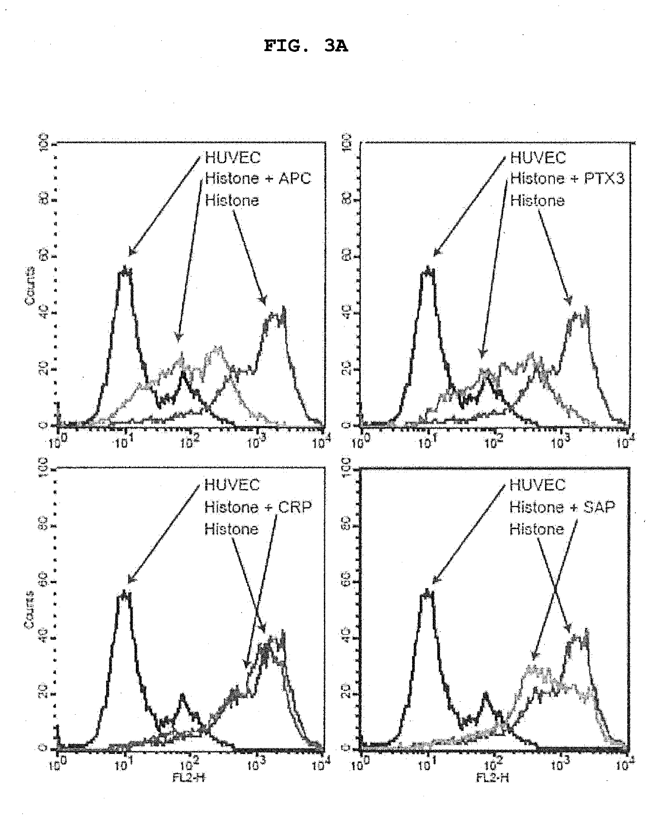

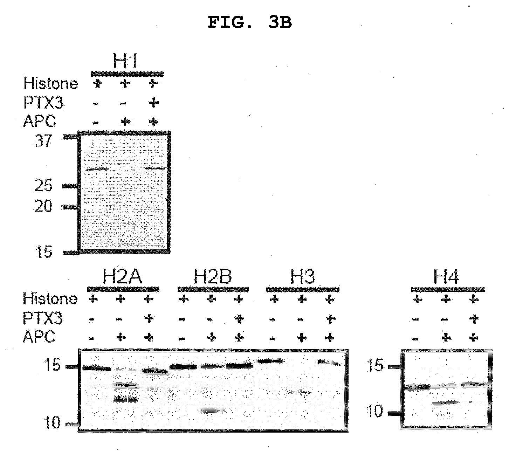

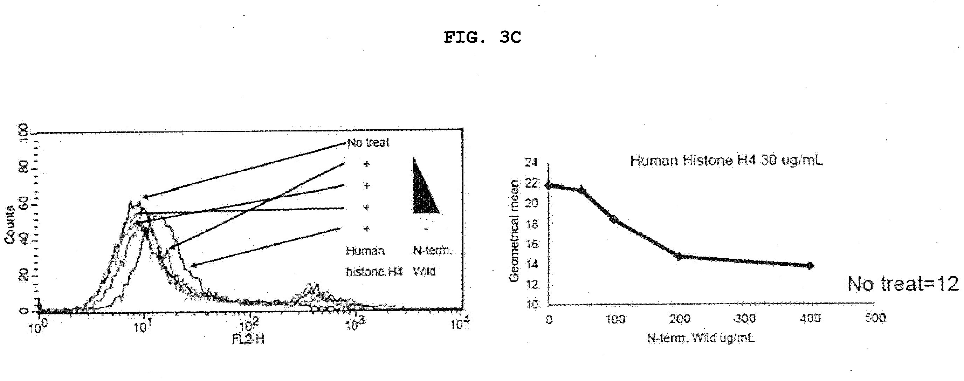

The relationship between the PTX3-histone aggregation reaction found this time and the cytotoxic activity possessed by extracellular histone was examined. As a result, it was demonstrated that PTX3 suppresses cytotoxic activity of extracellular histone on HUVEC to the same level as APC, and CRP and SAP do not have such suppressive effect (Fig. 3A). The relationship between the decomposition activity of APC on histone and the aggregation capacity of PTX3 was confirmed. As a result, PTX3-histone aggregate was found to be resistant to decomposition by APC (Fig. 3B). In addition, the N-terminal domain of PTX3 (18 - 178 amino acids) was also confirmed to suppress, like PTX3 (full-length), cytotoxic activity of histone on HUVEC (Fig. 3C).

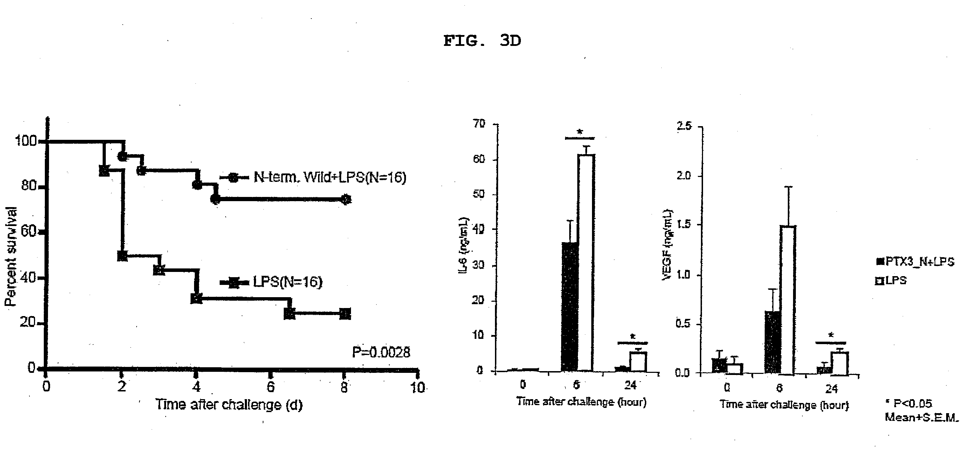

Such PTX3-histone aggregation reaction and the effect of suppression of vascular endothelial cytotoxic activity of histone by PTX3 were studied in vivo. For the study, the PTX3 N-terminal domain involved in the aggregation with histone was used. The mouse administered with the N-terminal domain of PTX3 showed a significantly improved mortality due to LPS administration (Fig. 3D). In addition, biochemical study of blood revealed that inflammatory reaction caused by LPS administration was suppressed in the mouse administered with the N-terminal domain of PTX3 (Fig. 3D).

From the above-mentioned results, it was considered that the N-terminal domain of PTX3 causes an aggregation reaction with extracellular histone, which is a malignant factor for the living organisms, and affords an effect to neutralize histone in vivo. Therefrom it was considered that the N-terminal domain of PTX3 is useful for the treatment or prophylaxis of diseases associated with extracellular histone.

### 4. Binding activity and affinity between histone variant and PTX3

The binding level between immobilized each recombinant histone and tag-free recombinant human PTX3 (rhPTX3) was measured by binding assay (ELISA). For the detection, HRP-labeled anti-PTX3 monoclonal antibody (PPZ1228) was used. ELISA was performed with duplicate wells for each point, and means and standard deviations were calculated from three independent experiments.

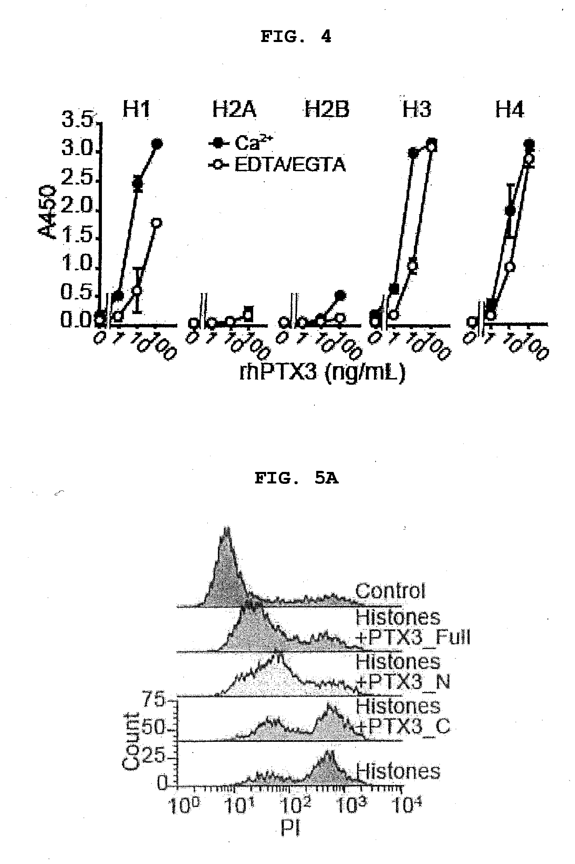

As a result, different binding property was shown for each histone variant (Fig. 4). Particularly, histones H1, H3 and H4 showed strong binding affinity for PTX3. While the binding of PTX3 and each histone did not require calcium ion, stronger binding was found in the presence of calcium ion.

The binding level between immobilized each recombinant histone and a tagged human PTX3 fragment was measured by binding assay (ELISA). For detection, HRP-labeled anti-His (6xHis) antibody was used. ELISA was performed with duplicate wells for each point, and means and standard deviations were calculated from three independent experiments.

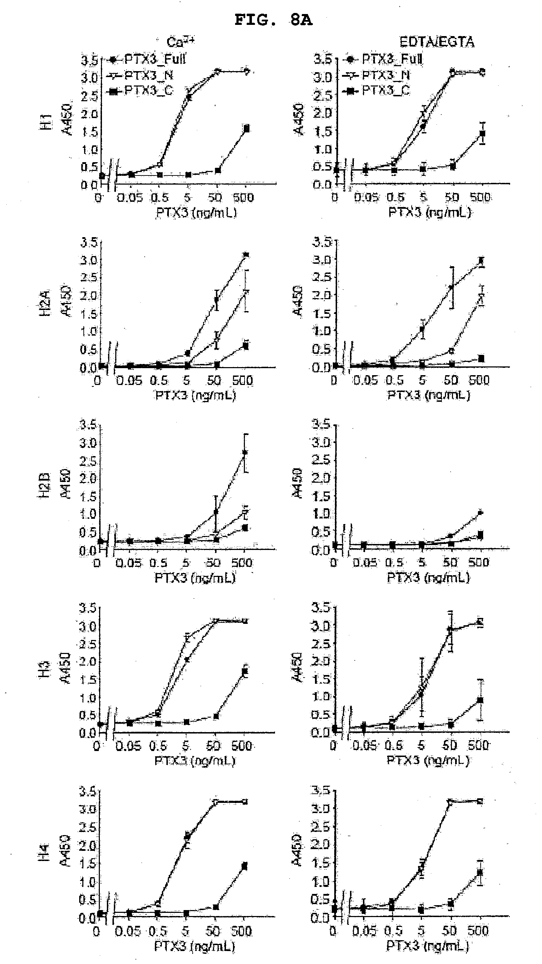

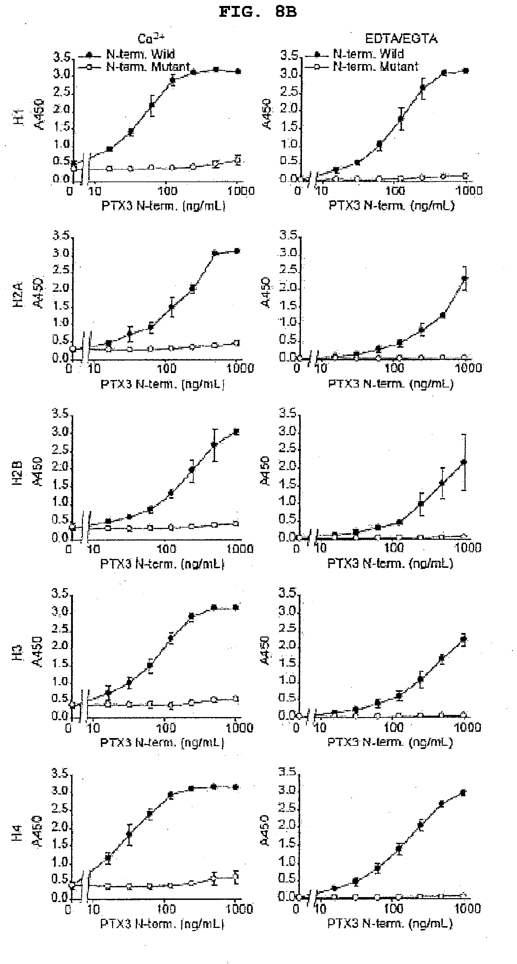

As a result, the N-terminal domain of PTX3 showed bindability to histones H1, H3 and H4, which was equivalent to that of the full-length PTX3 (Fig. 8A). The bindability of the N-terminal domain of PTX3 to histones H2A and H2B was lower than that of the full-length PTX3. While the binding of the N-terminal domain of PTX3 to each histone did not require calcium ion, stronger binding was found in the presence of calcium ion (Fig. 8A). Although weak, the C-terminal domain of PTX3 also showed bindability to each histone (Fig. 8B). The N-terminal domain that lost the oligomer formation capacity by replacing the 47th, 49th, 103rd cysteine residues with serine residue was scarcely bound to each histone (Fig. 8B).

The binding of immobilized each recombinant histone, and Myc- and His-tagged human PTX3 fragment was measured by surface plasmon resonance (SPR). The highest concentration of each PTX3 was as follows. full-length PTX3: 1 nM, N-terminal domain of PTX3: 5 nM, C-terminal domain of PTX3: 50 nM. The assay buffer contained 4 mM CaCl₂ (Ca²⁺) or 4 mM EDTA/EGTA. The affinity measured by SPR was calculated from three experiments.

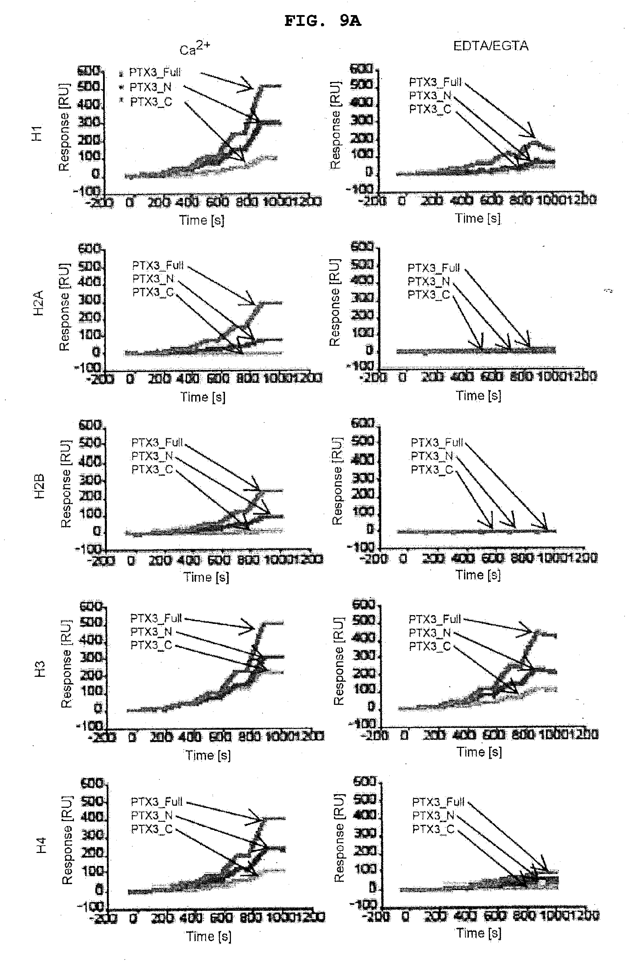

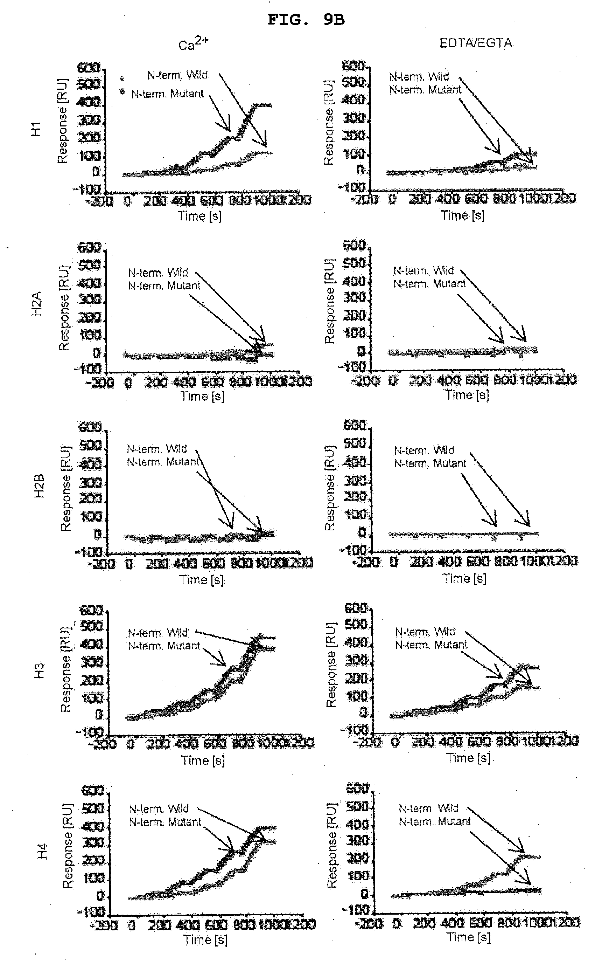

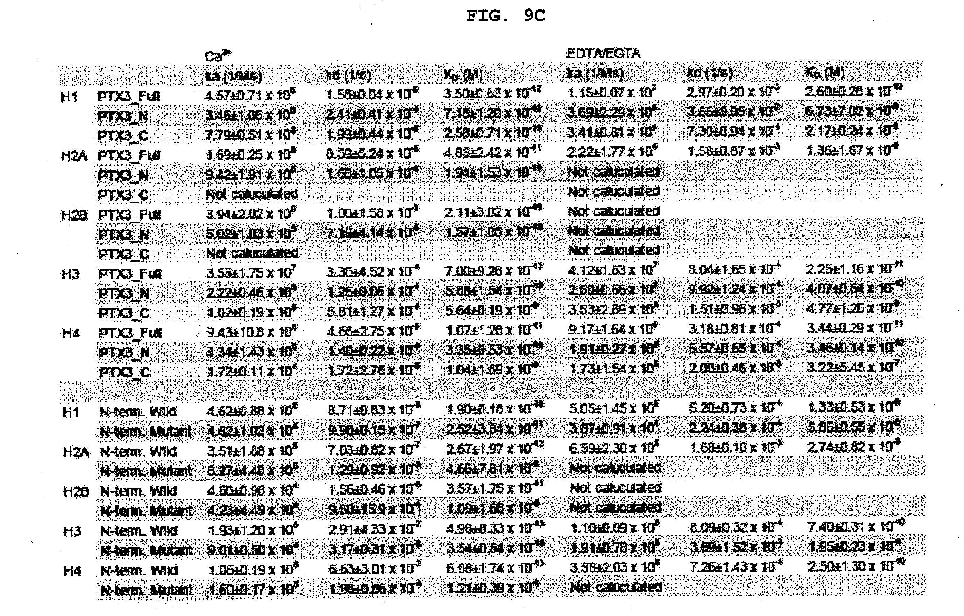

As a result, in SPR, the affinity for each histone was higher in the order of full-length PTX3> N-terminal domain> C-terminal domain (Figs. 9A and C). By EDTA/EGTA treatment, the affinity for each histone was attenuated (Figs. 9A and C). The N-terminal domain that lost the oligomer formation capacity by replacing the 47th, 49th, 103rd cysteine residues with serine residue showed bindability but the affinity decreased as compared to the wild-type (Fig. 9B and C). By EDTA/EGTA treatment, the affinity for each histone was attenuated (Figs. 9B and C).

### 5. Suppressive effect of N-terminal domain of PTX3 on histone cytotoxicity

HUVECs were cultured in a medium concurrently containing Calf Thymus Histones (100 µg/mL), and PTX3 fragment (40 µg/mL), CRP (40 µg/mL) or SAP (40 µg/mL) at 37°C for 1 hr, and the level of cytotoxic activity was measured using FACS. The histone cytotoxicity on HUVEC was evaluated by propidium iodide (PI) staining.

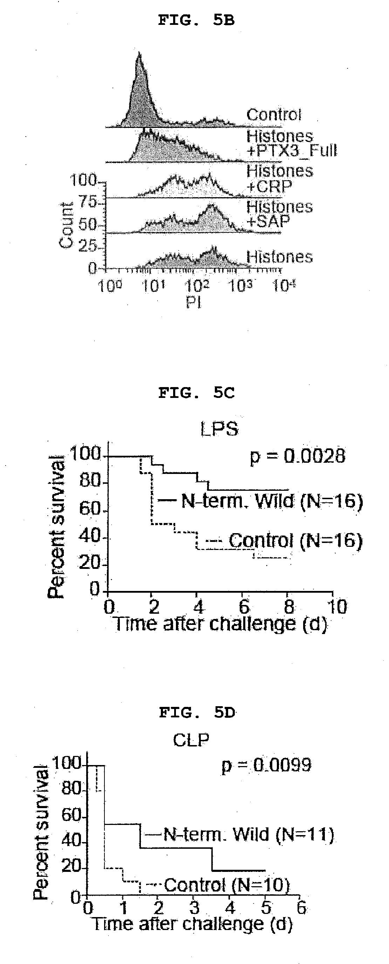

As a result, it was demonstrated that the full-length PTX3 and the N-terminal domain of PTX3 suppress toxic activity of histone on HUVEC, and CRP and SAP do not have such suppressive effect (Figs. 5A and B).

Using each recombinant histone instead of Calf Thymus Histones, a suppressive effect of full-length PTX3 or the N-terminal domain of PTX3 on histone cytotoxicity was studied in the same manner.

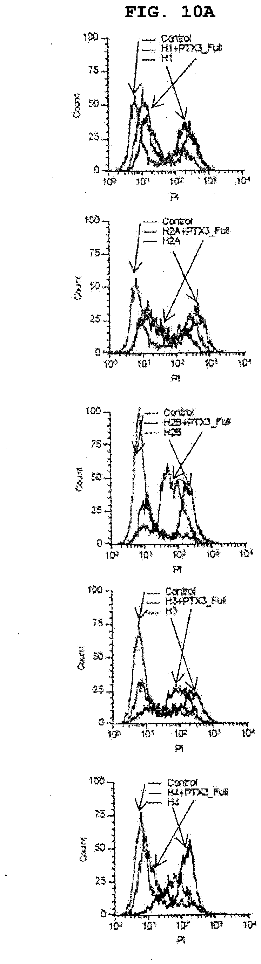

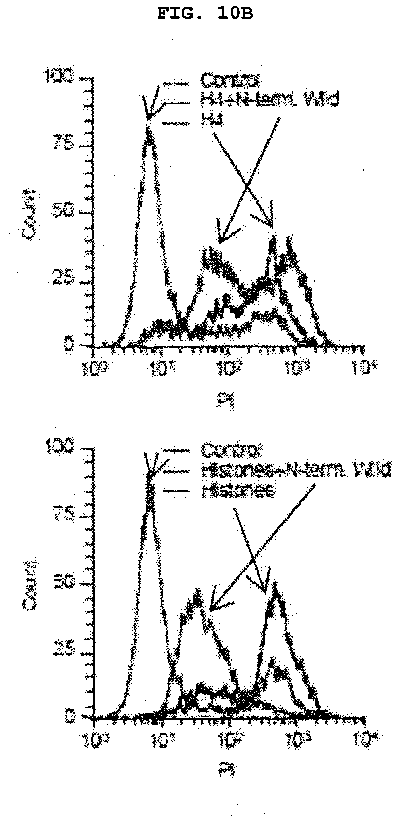

As a result, the full-length PTX3 suppressed the toxic activity of each recombinant histone on HUVEC (Fig. 10A). The N-terminal domain of PTX3 suppressed the toxic activity of histone H4 and Calf Thymus Histones on HUVEC (Fig. 10B).

The survival rate of the mice intraperitoneally administered with LPS at a concentration of 16 mg/kg is shown. The N-terminal domain of PTX3 (5 mg/kg) or control buffer was intraperitoneally administered 2 hr prior to LPS administration. In addition, the time-course changes in the plasma IL-6 and VEGF levels of the LPS-administrated mice treated with the N-terminal domain of PTX3 were measured by ELISA.

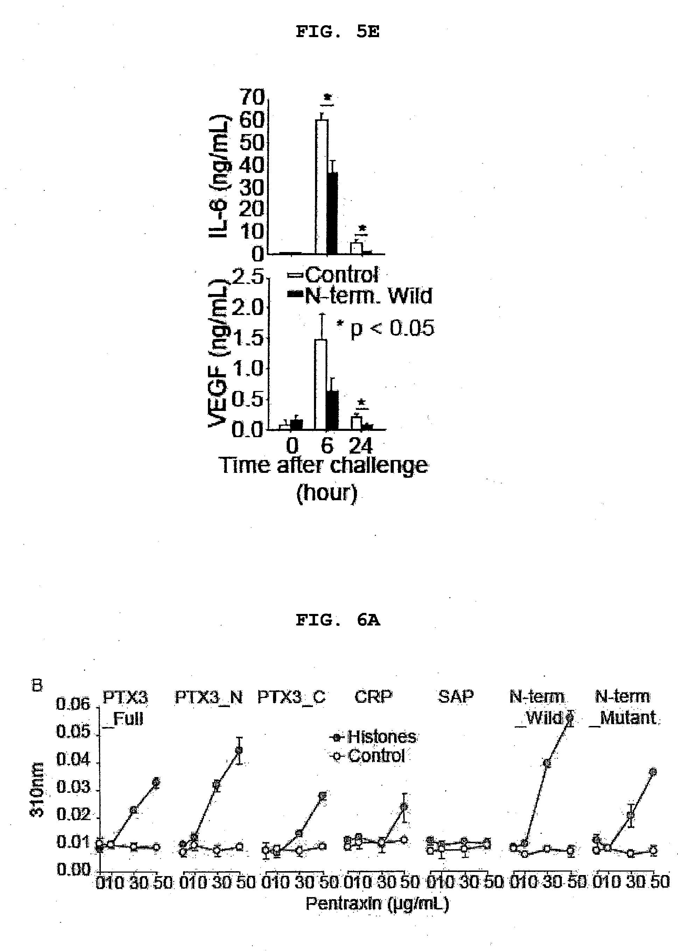

As a result, the mortality due to LPS administration was significantly improved in the mice administered with the N-terminal domain of PTX3 (Fig. 5C). In addition, administration of the N-terminal domain of PTX3 suppressed the plasma IL-6 and VEGF levels (Fig. 5E). From these results, it was shown that the mice administered with the N-terminal domain of PTX3 were resistant to lethality of LPS administration and inflammatory reaction due to LPS administration was suppressed.

Mouse was subjected to cecal ligation and puncture (CLP) treatment and, 4 hr later from the treatment, the N-terminal domain of PTX3 (5 mg/kg) and gentamicin (5 mg/kg), or gentamicin (5 mg/kg) alone were(was) administered to the mouse.

As a result, mortality due to the CLP treatment was significantly improved in the mouse administered with the N-terminal domain of PTX3 (Fig. 5D).

From the above-mentioned results, the N-terminal domain of PTX3 was considered to cause aggregation reaction with extracellular histone, which is a malignant factor for living organisms, and afford an effect of neutralizing histone in vivo. From this, it was considered that the N-terminal domain of PTX3 is useful for the treatment or prophylaxis of diseases associated with extracellular histone. The suppressive effect on the cytotoxicity of histone variant is shown in Fig. 10.

### 6. Aggregation mechanism of histone and PTX3

The aggregation activity of PTX3 and other pentraxins to histone was evaluated. Calf Thymus Histones (50 µg/mL) were mixed with a PTX3 fragment or the other pentraxin at each concentration, and the absorbance at the wavelength of 310 nm was measured. Before mixing, samples were dialyzed against 4 mM CaCl₂-containing TBS.

As a result, the full-length PTX3 and N-terminal domain of PTX3 showed an aggregation activity with histone (Fig. 6A). While the C-terminal domain of PTX3 also showed a certain level of aggregation activity with histone, the activity was weaker than that of the full-length PTX3 and N-terminal domain of PTX3 (Fig. 6A). CRP and SAP scarcely showed an aggregation activity (Fig. 6A). The N-terminal domain that lost the oligomer formation capacity by replacing the 47th, 49th, 103rd cysteine residues with serine residue showed a decreased aggregation activity.

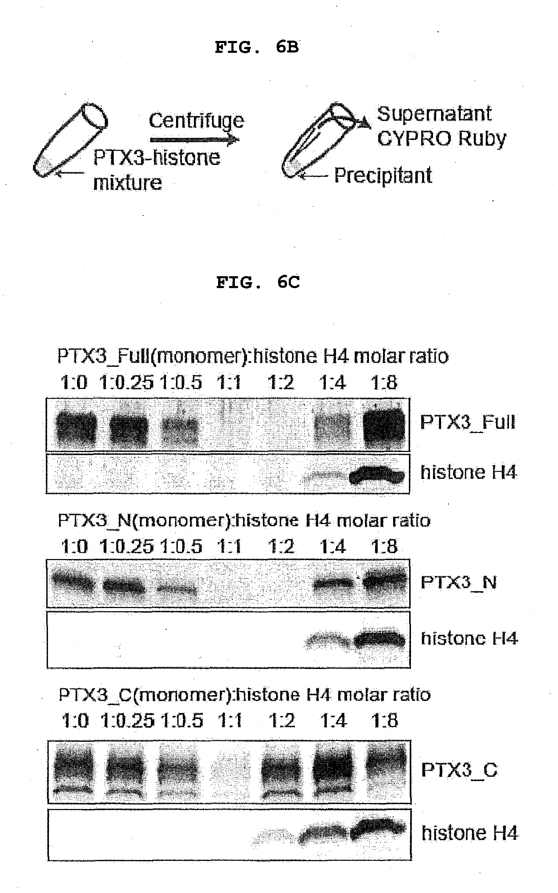

The aggregation stoichiometry was studied. Each PTX3 fragment (50 µg/mL) was mixed with recombinant histone H4 having various molar ratios, the aggregate was removed by centrifugation, PTX3 and histone H4 remaining in the supernatant were recovered and detected by SYPRO Ruby staining (Fig. 6B).

As a result, it was shown that the full-length PTX3 and N-terminal domain of PTX3 aggregate with histone H4 at a molar ratio of 1:1 - 1:2 (PTX3 (or N-terminal domain):histone H4) (Fig. 6C). On the other hand, it was shown that the C-terminal domain of PTX3 aggregate with histone H4 at a molar ratio of 1:1 - 1:2 (PTX3 C-terminal domain:histone H4) (Fig. 6C).

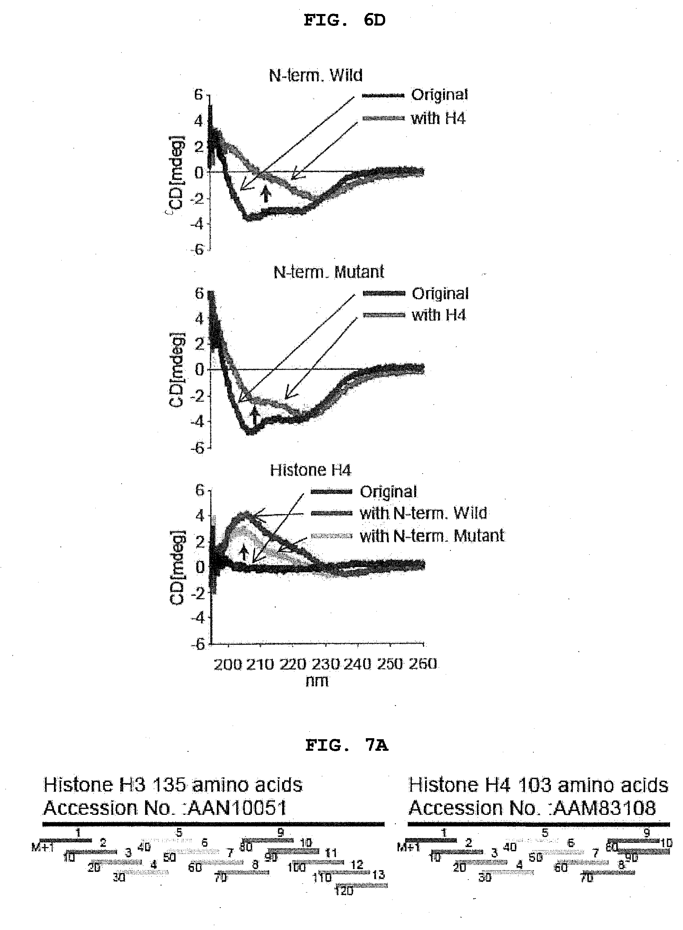

The CD spectrum of the histone H4-PTX3 complex was measured. The N-terminal domain of PTX3 (25 µg/mL) and histone H4 (5 µg/mL) were each independently measured, and further, the histone H4-PTX3 mixture was measured (Fig. 6D).

As a result, when the N-terminal domain of PTX3 alone was used, a spectrum pattern having α-helix in the secondary structure was obtained (Fig. 6D upper and middle "Original"), whereas the N-terminal domain of PTX3 mixed with histone H4 did not afford a spectrum pattern showing a general secondary structure (Fig. 6D upper and middle "with H4"). Similarly, histone H4 mixed with the N-terminal domain of PTX3 did not afford a spectrum pattern showing a general secondary structure (Fig. 6D lower).

From these results, it was considered that both histone H4 and PTX3 have an unstable secondary structure due to the histone H4-PTX3 complex formation, thereby suggesting that the unstable structure is another reason to form an aggregate.

### 7. Suppressive effect of PTX3 on cytotoxic activity of histone H3 and H4 fragments

The suppressive effect of PTX3 on cytotoxic activity of histone H3 and H4 fragments was evaluated. As the histone H3 and H4 fragments, those shown in Fig. 7A and Table 1 were used.

**Table 1**

| Peptide | Sequence | Residues |
|---|---|---|
| H3-1 | MARTKQTARKSTGGKAPRKQ (SEQ ID NO:4) | M+1-19 |
| H3-2 | STGGKAPRKQLATKAARKSA (SEQ ID NO:5) | 10-29 |
| H3-3 | LATKAARKSAPATGGVKKPH (SEQ ID NO:6) | 20-39 |
| H3-4 | PATGGVKKPHRYRPGTVALR (SEQ ID NO:7) | 30-49 |
| H3-5 | RYRPGTVALREIRRYQKSTE (SEQ ID NO:8) | 40-59 |
| H3-6 | EIRRYQKSTELLIRKLPFQR (SEQ ID NO:9) | 50-69 |
| H3-7 | LLIRKLPFQRLVREIAQDFK (SEQ ID NO:10) | 60-73 |
| H3-8 | LVREIAQDFKTDLRFQSSAV (SEQ ID NO:11) | 70-89 |
| H3-9 | TDLRFQSSAVMALQEACEAY (SEQ ID NO:12) | 80-99 |
| H3-10 | MALQEACEAYLVGLFEDTNL (SEQ ID NO:13) | 90-109 |
| H3-11 | LVGLFEDTNLCAIHAKRVTI (SEQ ID NO:14) | 100-119 |
| H3-12 | CAIHAKRVTIMPKDIQLARR (SEQ ID NO:15) | 110-129 |
| H3-13 | MPKDIQLARRIRGERA (SEQ ID NO:16) | 120-135 |
| H4-1 | MSGRGKGGKGLGKGGAKRHR (SEQ ID NO:17) | M+1-19 |
| H4-2 | LGKGGAKRHRKVLRDNIQGI (SEQ ID NO:18) | 10-29 |
| H4-3 | KVLRDNIQGITKPAIRRLAR (SEQ ID NO:19) | 20-39 |
| H4-4 | TKPAIRRLARRGGVKRISGL (SEQ ID NO:20) | 30-49 |
| H4-5 | RGGVKRISGLIYEETRGVLK (SEQ ID NO:21) | 40-59 |
| H4-6 | IYEETRGVLKVFLENVIRDA (SEQ ID NO:22) | 50-69 |
| H4-7 | VFLENVIRDAVTYTEHAKRK (SEQ ID NO:23) | 60-79 |
| H4-8 | VTYTEHAKRKTVTAMDVVYA (SEQ ID NO:24) | 70-89 |
| H4-9 | TVTAMDVVYALKRQGRTLYG (SEQ ID NO:25) | 80-99 |
| H4-10 | LKRQGRTLYGFGG (SEQ ID NO:26) | 90-103 |

HUVEC was cultured in a medium added with each fragment (250 µg/mL) at 37°C for 1 hr, and the level of toxic activity was measured using FACS. For evaluation of cytotoxicity, propidium iodide (PI) staining was used.

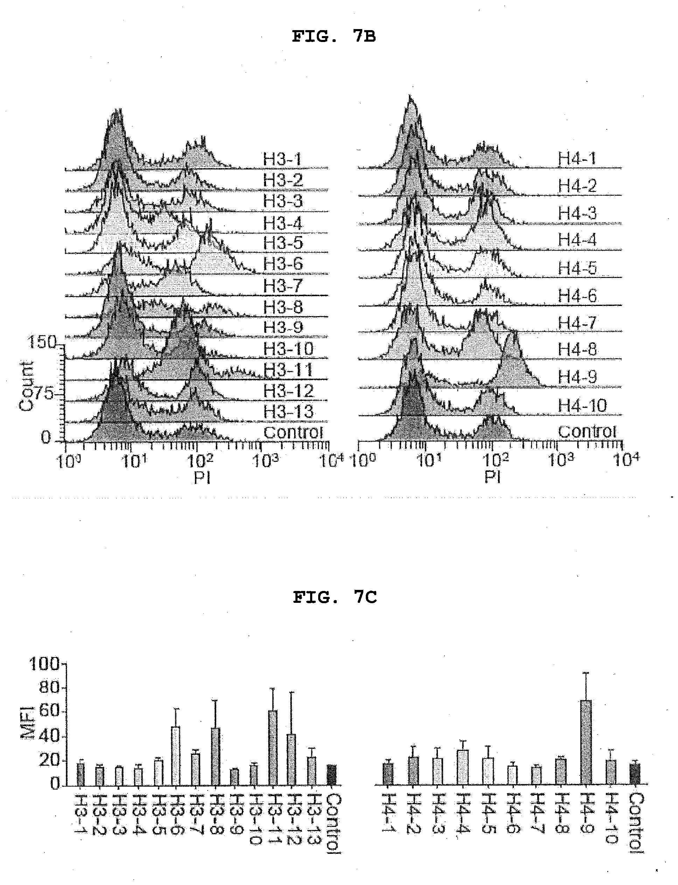

As a result, fragments H3-6, H3-8, H3-11 and H4-9 showed cytotoxic activity (Figs. 7B, 7C).

Then, a suppressive effect of PTX3 on the cytotoxic activity of fragments H3-6, H3-8, H3-11 and H4-9 was evaluated. Each fragment (250 µg/mL) was added to a medium containing or without containing full-length PTX3 (80 µg/mL), HUVEC was cultured at 37°C for 1 hr and the level of toxic activity was measured by FACS. For evaluation of cytotoxicity, propidium iodide (PI) staining was used.

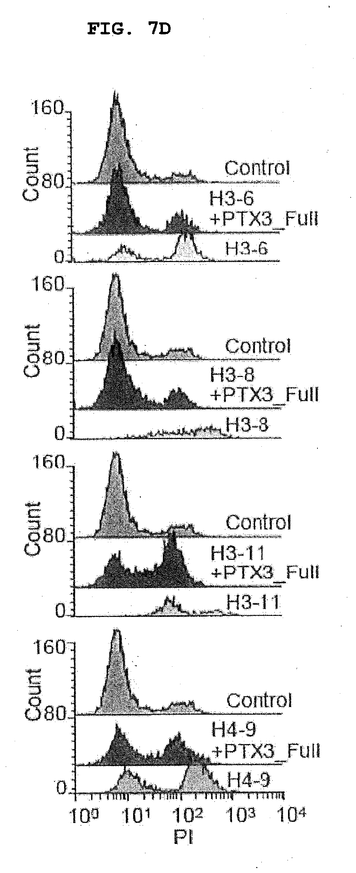

As a result, PTX3 neutralized the cytotoxic activity of fragments H3-6, H3-8, H3-11 and H4-9 (Fig. 7D). From these results, it was suggested that PTX3 neutralizes the cytotoxic activity by binding to the cytotoxicity active center of histones H3 and H4.

### 8. Analysis results of aggregation of histone and PTX3

Full-length PTX3 at each concentration was mixed with 50 µg/mL histone H4, and UV-visible absorption spectrum (wavelength 310 nm) was measured.

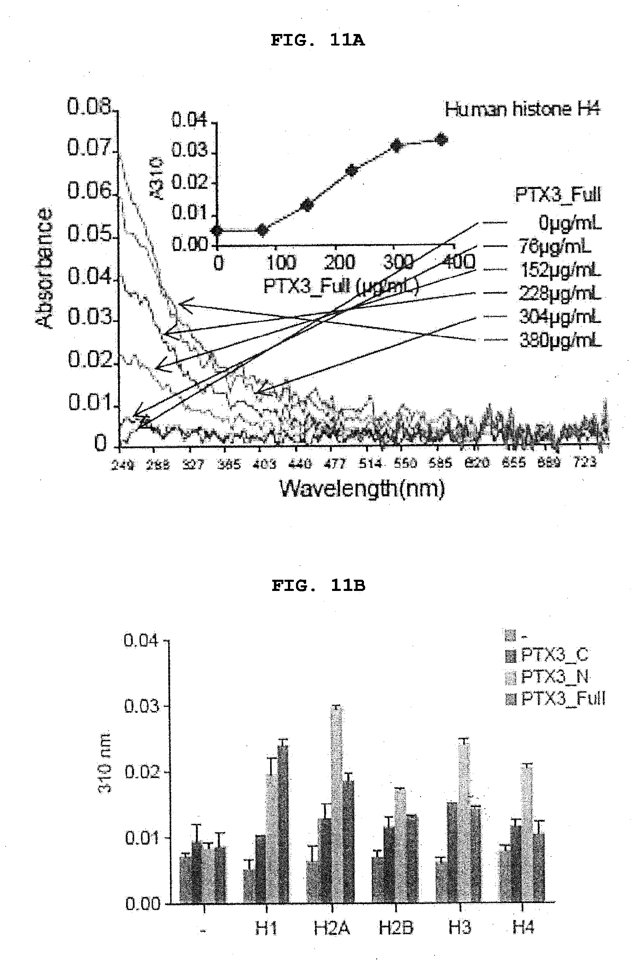

As a result, an aggregate-like substance was confirmed in a mixture of PTX3 and histone by visual observation, and a dose-dependent spectrum was observed in the measurement of UV-visible absorption spectrum (Fig. 11A). Since an increase in the UV-visible absorption spectrum is not observed in general protein binding, it was clarified that an aggregation reaction took place between PTX3 and histone. The spectrum shown in Fig. 11A was considered to have been caused by the scattering of the aggregates including the both.

Each histone variant (50 µg/mL) and each PTX3 fragment (30 µg/mL) were mixed, and the absorbance at the wavelength of 310 nm was measured. Before mixing, samples were dialyzed against 4 mM CaCl₂-containing TBS.

As a result, full-length PTX3 and PTX3 N-terminal fragment aggregated with each histone variant (Fig. 11B). The C-terminal fragment of PTX3 also aggregated with each histone variant (Fig. 11B).

Full-length PTX3 at each concentration (0, 12.5, 25, 50 µg/mL) was mixed with Calf Thymus Histones (50 µg/mL) or histone H4 (50 µg/mL), and the mixture was centrifuged. The obtained supernatant was subjected to immunoblotting using PPZ-1228 or SYPRO Ruby staining.

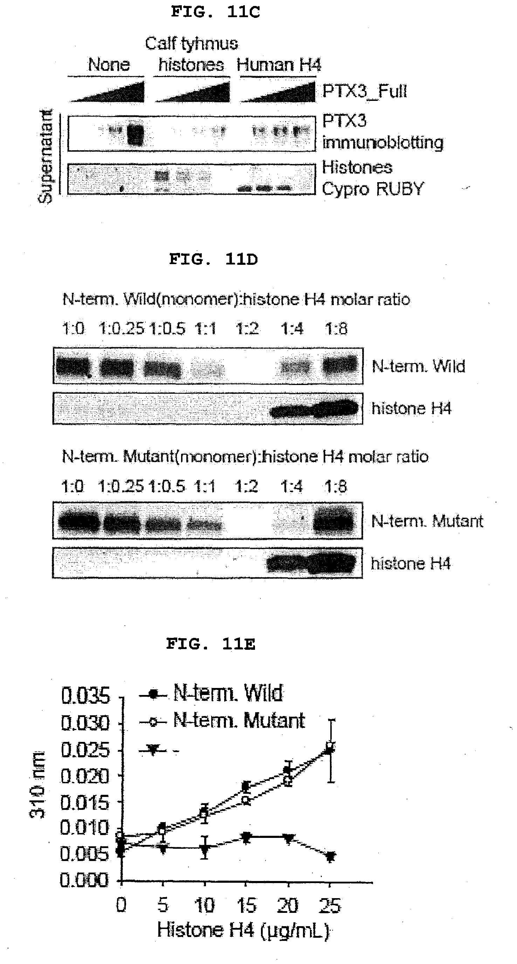

As a result, the higher the amount of PTX3 to be added was, the lower the contents of Calf Thymus Histones and histone H4 in the supernatant were (Fig. 11C). From these results, it was suggested that full-length PTX3 bound to Calf Thymus Histones and histone H4 and precipitated as an insoluble product.

The stoichiometry of aggregation of histone and the N-terminal domain of PTX3 was studied. Recombinant histone H4 at each concentration was mixed with wild-type (50 µg/mL) or mutant deficient in oligomer formation capacity (50 µg/mL) of the N-terminal domain of PTX3, the aggregate was removed, and residual PTX3 and histone H4 were detected by SYPRO Ruby staining.

As a result, the wild-type N-terminal domain and mutant N-terminal domain of PTX3 aggregated with histone H4 at a molar ratio of 1:2 (N-terminal domain:histone H4) (Fig. 11D).

Since no aggregation of sample during the measurement of CD spectrum is essential, histone H4 and PTX3 N-terminal domain (30 µg/mL) were mixed, and the concentration range of histone H4 free of an increase in the absorbance at a wavelength of 310 nm was confirmed. Prior to mixing, samples were dialyzed against 4 mM CaCl₂-containing TBS.

As a result, since an increase in the absorbance was scarcely found at a histone H4 concentration of 5 µg/mL, this concentration was used for the CD spectrum measurement (Fig. 11E).

### 9. PTX3 binding capacity of histone H3 and H4 fragments

The binding activity of each fragment of histone H3 and H4 (H3-1 - H3-13, and H4-1 - H4-10) to PTX3 was evaluated. Histone H3 and H4 and fragments thereof were spotted on a nitrocellulose membrane, stained with SYPRO Ruby, and reacted with PTX3 fragment. PTX3 was detected using an anti-myc antibody as a secondary reaction and a peroxidase-labeled F(ab')₂ fragment goat anti-mouse IgG(H+L) antibody as a tertiary reaction.

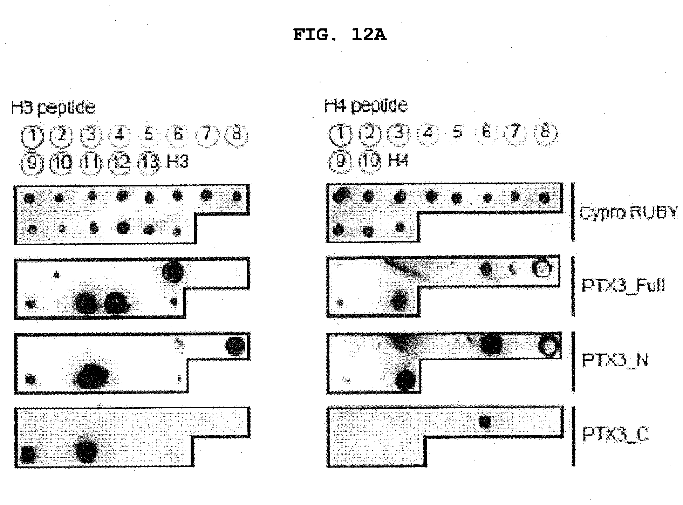

As a result, full-length PTX3 was bound to H3-2, 6, 8, 9, 11 and 12, the N-terminal of PTX3 was bound to H3-6, 8, 9 and 11, and the C-terminal fragment of PTX3 was bound to H3-9 and 11 (Fig. 12A left). Moreover, full-length PTX3 was bound to H4-6, 7, 8 and 9, N-terminal fragment of PTX3 was bound to H4-6, 8 and 9, and C-terminal fragment of PTX3 was bound to 4-6 (Fig. 12A right).

In the same manner as in Figs. 7B - D, a suppressive effect of the N-terminal domain of PTX3 on the cytotoxic activity of histone H3 and H4 fragments was evaluated.

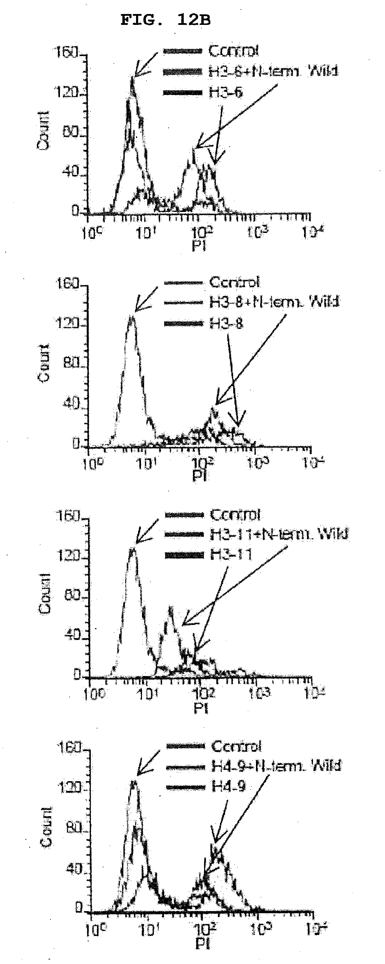

As a result, the N-terminal domain of PTX3 neutralized the cytotoxic activity of fragments H3-6, H3-8, H3-11 and H4-9 (Fig. 12B).

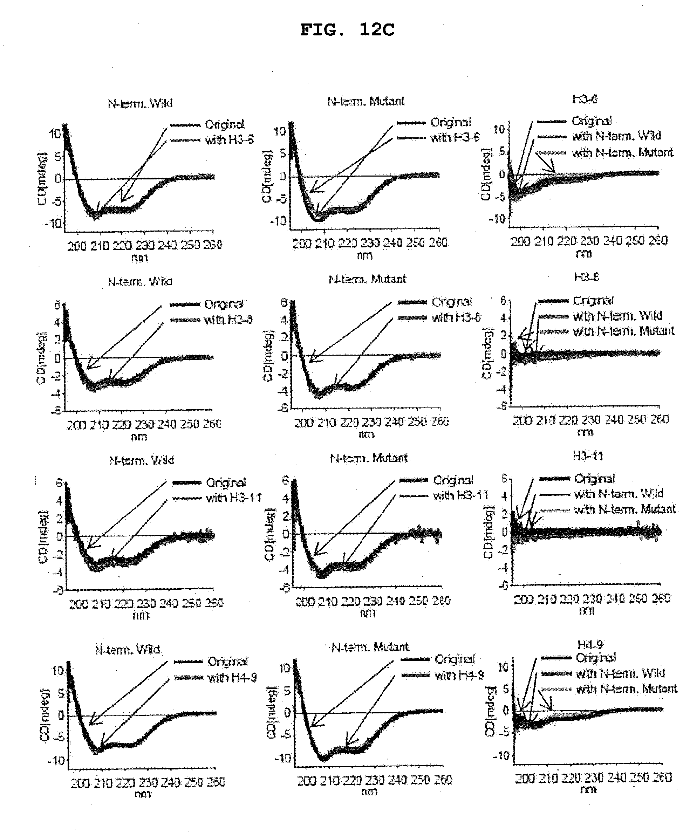

The CD spectrum of complexes of histone H3 and H4 fragments and PTX3 were measured. The N-terminal domain of PTX3 and histone H3 and H4 fragments (H3-6, H3-8, H3-11 and H4-9) were each independently measured, and a mixture of PTX3 and each fragment was further measured (Fig. 12C). The concentrations of the both were PTX3 (50 µg/mL) and H3-6 or H4-9 (50 µg/mL), PTX3 (25 µg/mL) and H3-8 or H4-11 (5 µg/mL).

As a result, changes in the secondary structure of the N-terminal domain of PTX3 due to the complex formation were not observed in any fragments (Fig. 12C). From these results, it was suggested that changes in the secondary structure of PTX3 due to the binding with histone are insufficient only by binding with a region of histone having cytotoxic activity.

### Experimental Example 6 Quantification of Histone by PTX3 N-terminal domain-immobilized ELISA (capture: PTX3 N-terminal domain, detection: anti-Histone H3 antibody)

PTX3 N-terminal domain was adjusted to 1 pg/ml with 20 mM Carbonate (pH 9.6), dispensed to 96 well plate (Maxisorp Immunomodule (Thermo, #468667)) by 50 µL, and stood at 4°C overnight. The plate was washed, CE510 (0.1% (w/v)) was dispensed by 300 pL, and the plate was stood at 25°C for 2 hr. As histone samples, a bovine thymus-derived purified product (Histone from calf thymus, TypeII-A (Lyophilized powder), SIGMA H9250) and human Recombinant (Histone H3.1/H4 tetramer Human, Recombinant, BioLabs #M2509S) were used. The plate was washed, and shaken with Histone sample (0, 1, 3, 10, 30, 100, 300 ng/mL) (50 µL) adjusted with 20 mM Carbonate, pH 9.6, 0.8M NaCl, 1% BSA at 25°C for 1 hr. The plate was washed, and shaken with a 5,000-fold diluted solution (50 µL) of anti-Histone H3 antibody (Anti-Histone H3, mouse monoclonal antibody, (Clone No MABI0301), Wako 300-34783) at 25°C for 1 hr. The plate was washed, and a 5,000-fold diluted solution (50 µL) of Anti-Mouse IgG Peroxidase conjugate (SIGMA A3673) was shaken at 25°C for 30 min. The plate was washed, a substrate solution (1-Step Ultra TMB - ELISA (Thermo, #34028) (50 µL) was reacted at 25°C and, 30 min later, the reaction stop solution (50 µL) was dispensed. The absorbance (450 nm) was measured by a plate reader (ARVO, PerkinElmer).

The results are shown in Table 2.

**Table 2**

| calf thymus -derived Histone | |
|---|---|
| ng/ml | absorbance |
| 0 | 0.041 |
| 1 | 0.047 |
| 3 | 0.058 |
| 10 | 0.105 |
| 30 | 0.286 |
| 100 | 1.186 |
| 300 | 2.127 |

| human Recombinant Histone | |
|---|---|
| ng/ml | absorbance |
| 0 | 0.044 |
| 1 | 0.047 |
| 3 | 0.053 |
| 10 | 0.066 |
| 30 | 0.144 |
| 100 | 0.708 |
| 300 | 2.266 |

The quantified value of Histone by PTX3 N-terminal domain-immobilized ELISA using anti-Histone H3 antibody for the detection did not show much difference between the bovine thymus-derived purified product and human Recombinant. Good quantitativeness was observed in the region around 10 - 100 ng/ml in all Histones.

### Experimental Example 7 Identification of PTX3 N-terminal domain site for suppressing cytotoxic activity of extracellular histone

### (1) Construct, expression and purification of PTX3 N-terminal domain fragment

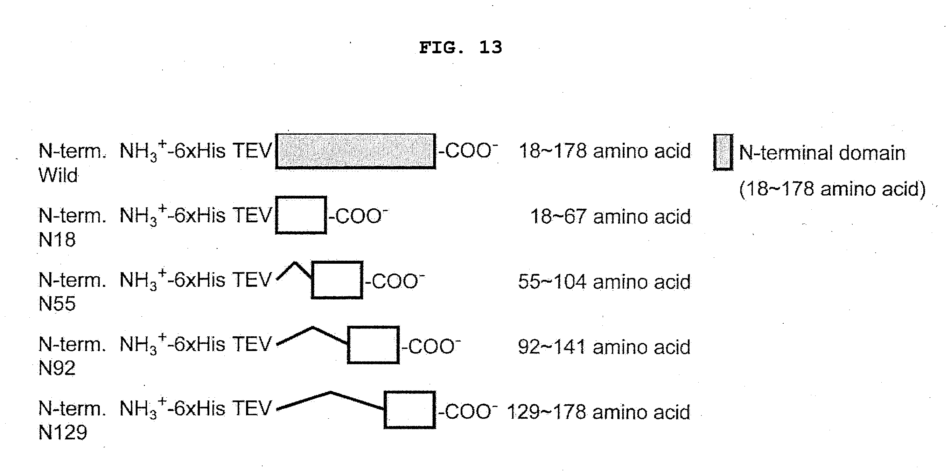

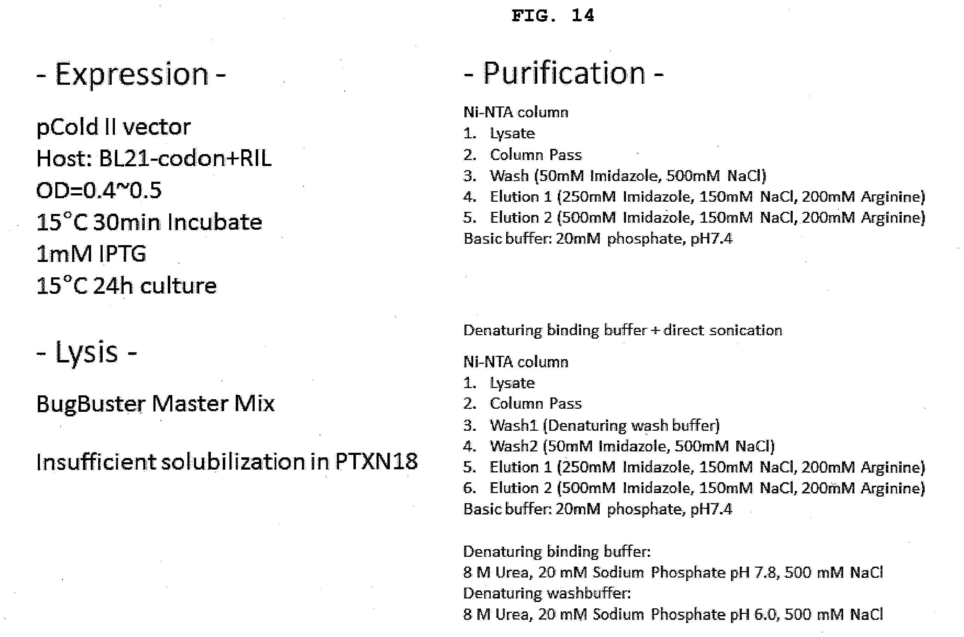

Obtainment of PTX3 N-terminal domain fragment was tried in an attempt to identify an important region in the PTX3 N-terminal domains, for binding and aggregating with histone, and suppressing cytotoxic activity. The PTX3 N-terminal domain was divided into four by about 50 amino acids, and expression constructs having His-tag and TEV sequence linked to the N-terminal side, as PTX3 N-terminal domain expressed in Escherichia coli used in Experimental Example 3, were produced (Fig. 13). First, in the same manner as with the PTX3 N-terminal domain expressed in Escherichia coli, each fragment was expressed in Escherichia coli by a conventional method, and Escherichia coli pellets were lysed in BugBuster and purified using Ni-NTA column (Fig. 14). As a result, with PTXN18, a large amount of insoluble fractions was produced on lysis in BugBuster, and protein was hardly obtained; however, protein could be obtained with other fragments. As for PTXN18, insoluble fractions were solubilized using 8M Urea and ultra sonication, trapped by Ni-NTA column in the presence of 8M Urea, washed, and washed and eluted with Urea-free buffer, whereby the protein could be obtained.

### (2) Quality check of PTX3 N-terminal domain fragments

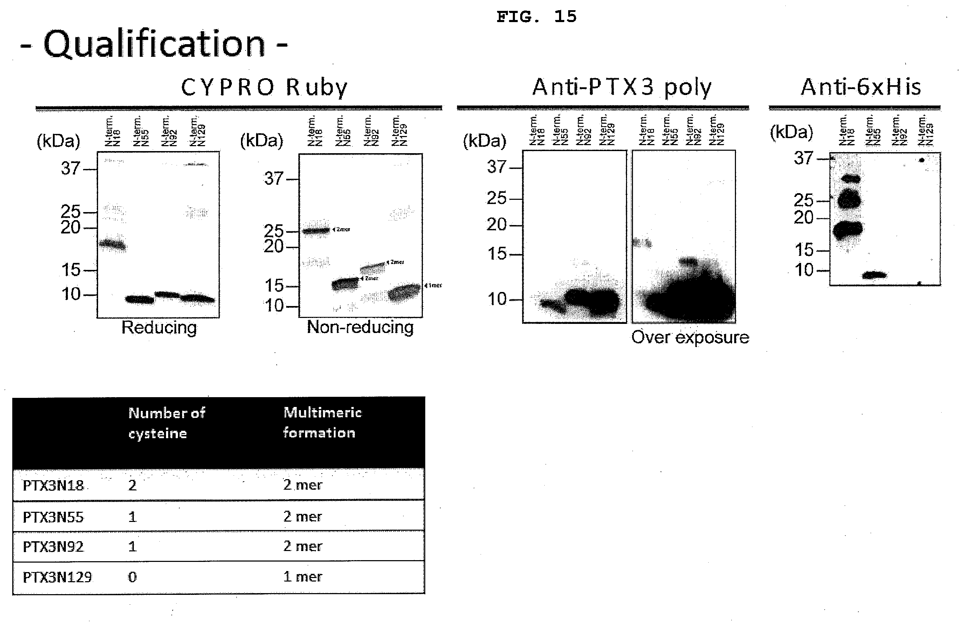

PTX3 N-terminal domain forms an oligomer (dimer and tetramer) via an S-S bond. However, oligomer formation capacity of a shorter fragment of N-terminal domain is unknown. Therefore, the molecular weight of each fragment under reducing/non-reducing conditions was confirmed by SDS-PAGE. Each protein was separated under reducing (2ME+) or nonreducing (2ME-) conditions by SDS-PAGE, and CYPRO Ruby protein staining was performed. As a result, in three fragment proteins having a cysteine residue, a stained band in a nonreducing state shifted to a molecular weight 2-fold that in a reducing state, and the rest of the fragments free of a cysteine residue showed no difference in the molecular weight between reducing/non-reducing (Fig. 15). From the above results, it was shown that the PTX3 N-terminal domain fragment proteins having a cysteine residue, which were obtained in this experiment, form a dimer via an S-S bond. The same samples were confirmed by Westernblot using a commercially available anti-PTX3 polyclonal antibody obtained using full-length PTX3 protein as an immunogen. As a result, the bands were detected in all fragment proteins (Fig. 15). In Westernblot using an anti-His-tag antibody, the band was not observed in PTXN92 and 129 (Fig. 15). While the reason for no reaction of the anti-His-tag antibody is unknown, the results of analysis performed using a mass spectrometry device raise a possibility of the lack of tag of the obtained protein. Therefore, it was assumed that the tag site was cleaved for some reason after the purification. While the predicted molecular weight of the fragment protein was 8 - 9 kDa, PTXN18 molecular weight was larger than the predicted molecular weight. The reason therefor is unknown.

### (3) Binding and aggregation of PTX3 N-terminal domain fragment with histone, and suppression of cytotoxic activity

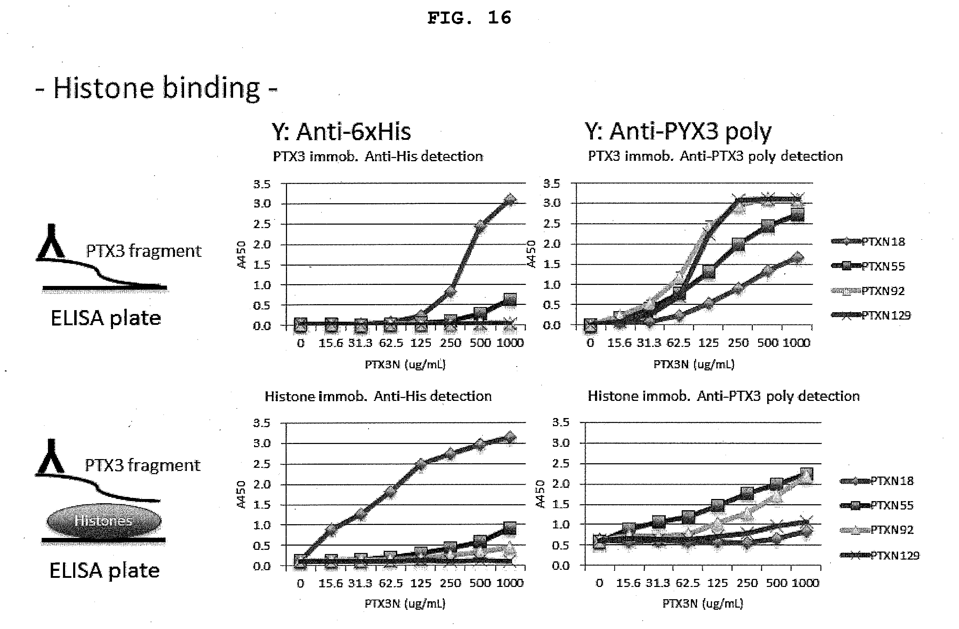

The bindability of the obtained fragment proteins and histone was confirmed by ELISA. For detection in ELISA, an anti-His-tag antibody has been used heretofore. As mentioned in (2), since some fragments do not react with this antibody, detection with an anti-PTX3 polyclonal antibody was also performed concurrently. Prior to the confirmation of bindability with histone, each fragment protein was first immobilized on an ELISA plate, and the reactivity with each antibody was confirmed. As a result, the reactivity varied in all antibodies, showing the same tendency with the Westernblot (Fig. 16, upper panel). Using the same antibody, bindability with histone was confirmed. As a result, a certain level of bindability with histone was seen in all fragments (Fig. 16, lower panel).

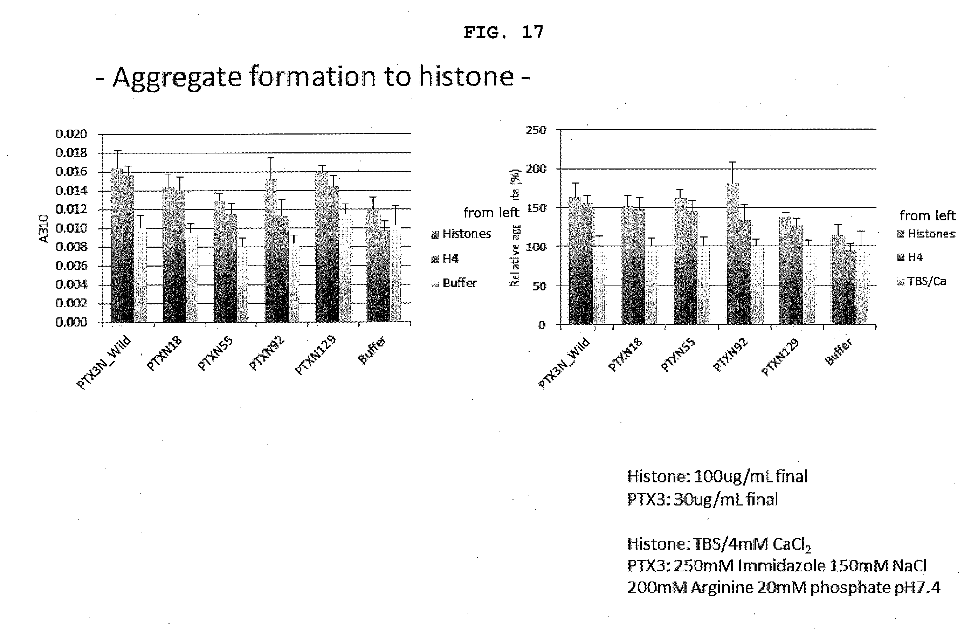

Then, aggregation with histone was confirmed by absorption at 310 nm. As a result, aggregation with histone was observed in all fragments (Fig. 17).

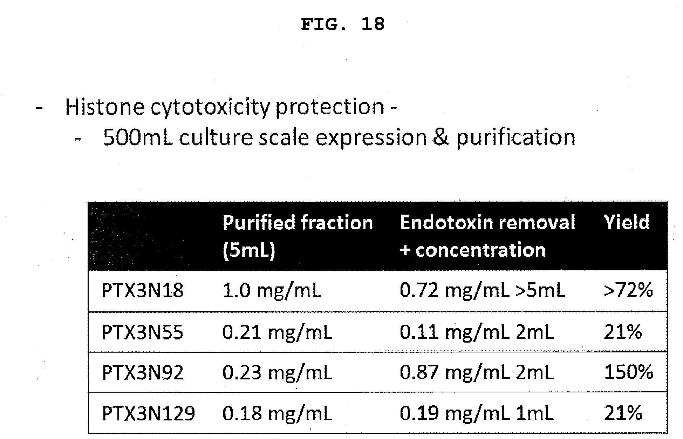

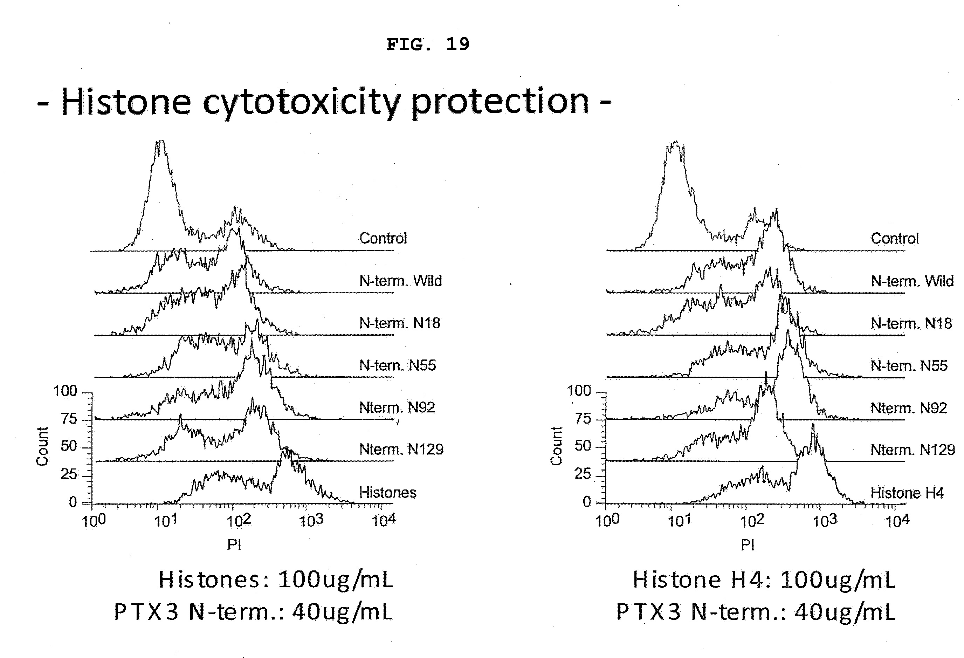

Lastly, capacity of suppressing the cytotoxic activity of histone was confirmed. Since this experiment requires a certain amount of protein and an endotoxin-elimination treatment, a protein was expressed and purified at a 500 mL culture scale, and subjected to an endotoxin-elimination treatment (Fig. 18). In the case of PTX3 N-terminal domain, about 10 mg was obtained at a 500 mL culture scale, and a loss by the endotoxin-elimination treatment was scarcely observed. With fragment proteins, some proteins showed a loss due to the endotoxin-elimination treatment at an acquisition amount of 1 - 5 mg. A capacity of fragment proteins obtained at a 500 mL scale on HUVEC to suppress histone cytotoxicity was studied. As a result, for bovine thymus-derived histone, PTXN18 showed suppressive capacity almost equivalent to that of the PTX3 N-terminal domain, then suppressive capacity was seen in the order of 55, 92, 129 (Fig. 19, left). For recombinant histone H4, PTXN18 and 129 showed suppressive capacity almost equivalent to that of the PTX3 N-terminal domain, and then suppressive capacity was seen in the order of 55, 92. In consideration of the experiments relating to the binding and aggregation, all fragments were considered to possibly have a certain level of suppressive capacity.

### Industrial Applicability

The therapeutic or prophylactic agent of the present invention is useful in the pharmaceutical field for the treatment or prophylaxis of systemic inflammatory response syndrome (SIRS), and the diagnostic reagent and judgment method of the present invention are useful in the field of diagnosis of SIRS. Also, the polypeptide complex of the present invention is useful as, for example, a research reagent for analyzing the interaction of PTX3 and histone in SIRS, as well as a positive control for the above-mentioned diagnostic reagent and the like.

## Claims

1. A polypeptide comprising
(ii) a continuous partial sequence of the amino acid sequence shown in SEQ ID NO: 3 having a length of not less than 8 amino acids; or
(iii) an amino acid sequence having not less than 70% identity with the amino acid sequence of (ii);
which is capable of binding to histone to form a polypeptide aggregate, or a pharmacologically acceptable salt thereof, for use for treating or preventing systemic inflammatory response syndrome, wherein the continuous partial sequence of (ii) comprises any of the following regions:
(1) a region consisting of the 1st - 50th amino acids of the amino acid sequence shown in SEQ ID NO: 3,
(2) a region consisting of the 38th - 87th amino acids of the amino acid sequence shown in SEQ ID NO: 3,
(3) a region consisting of the 75th - 124th amino acids of the amino acid sequence shown in SEQ ID NO: 3, and
(4) a region consisting of the 112th - 161st amino acids of the amino acid sequence shown in SEQ ID NO: 3, and
wherein the length of the polypeptide is not more than 100 amino acids.

2. Use of a polypeptide comprising
(ii) a continuous partial sequence of the amino acid sequence shown in SEQ ID NO: 3 having a length of not less than 8 amino acids; or
(iii) an amino acid sequence having not less than 70% identity with the amino acid sequence of (ii);
which is capable of binding to histone to form a polypeptide aggregate, or a pharmacologically acceptable salt thereof, for quantifying histone in vitro,
wherein the continuous partial sequence of (ii) comprises any of the following regions:
(1) a region consisting of the 1st - 50th amino acids of the amino acid sequence shown in SEQ ID NO: 3,
(2) a region consisting of the 38th - 87th amino acids of the amino acid sequence shown in SEQ ID NO: 3,
(3) a region consisting of the 75th - 124th amino acids of the amino acid sequence shown in SEQ ID NO: 3, and
(4) a region consisting of the 112th - 161st amino acids of the amino acid sequence shown in SEQ ID NO: 3, and
wherein the length of the polypeptide is not more than 100 amino acids.

3. The use according to claim 2, wherein the polypeptide or a pharmacologically acceptable salt thereof is immobilized on a solid phase carrier.

4. A method of quantifying histone, comprising
(1) a step of contacting a polypeptide comprising
(ii) a continuous partial sequence of the amino acid sequence shown in SEQ ID NO: 3 having a length of not less than 8 amino acids; or
(iii) an amino acid sequence having not less than 70% identity with the amino acid sequence of (ii);
which is capable of binding to histone to form a polypeptide aggregate, or a pharmacologically acceptable salt thereof with a histone-containing sample to form a polypeptide aggregate comprising the polypeptide or a pharmacologically acceptable salt thereof and histone, and
(2) a step of quantifying the polypeptide aggregate obtained in step (1),
wherein the continuous partial sequence of (ii) comprises any of the following regions:
(1) a region consisting of the 1st - 50th amino acids of the amino acid sequence shown in SEQ ID NO: 3,
(2) a region consisting of the 38th - 87th amino acids of the amino acid sequence shown in SEQ ID NO: 3,
(3) a region consisting of the 75th - 124th amino acids of the amino acid sequence shown in SEQ ID NO: 3, and
(4) a region consisting of the 112th - 161st amino acids of the amino acid sequence shown in SEQ ID NO: 3, and
wherein the length of the polypeptide is not more than 100 amino acids.

5. The method according to claim 4, wherein the histone contained in the polypeptide aggregate is quantified in step (2) by an immunological method by using an antibody that specifically recognizes histone.

6. A polypeptide complex comprising
(1) a polypeptide comprising
(ii) a continuous partial sequence of the amino acid sequence shown in SEQ ID NO: 3 having a length of not less than 8 amino acids; or
(iii) an amino acid sequence having not less than 70% identity with the amino acid sequence of (ii);
which is capable of binding to histone to form a polypeptide aggregate, or a pharmacologically acceptable salt thereof, and (2) histone or a pentraxin 3 N-terminal domain-binding fragment thereof,
wherein the continuous partial sequence of (ii) comprises any of the following regions:
(1) a region consisting of the 1st - 50th amino acids of the amino acid sequence shown in SEQ ID NO: 3,
(2) a region consisting of the 38th - 87th amino acids of the amino acid sequence shown in SEQ ID NO: 3,
(3) a region consisting of the 75th - 124th amino acids of the amino acid sequence shown in SEQ ID NO: 3, and
(4) a region consisting of the 112th - 161st amino acids of the amino acid sequence shown in SEQ ID NO: 3, and
wherein the length of the polypeptide is not more than 100 amino acids.

7. The polypeptide complex according to claim 6, which is a polypeptide aggregate.

8. A method of producing a polypeptide complex comprising
(1) a polypeptide comprising
(ii) a continuous partial sequence of the amino acid sequence shown in SEQ ID NO: 3 having a length of not less than 8 amino acids; or
(iii) an amino acid sequence having not less than 70% identity with the amino acid sequence of (ii);
which is capable of binding to histone to form a polypeptide aggregate, or a pharmacologically acceptable salt thereof, and
(2) histone or a pentraxin 3 N-terminal domain-binding fragment thereof, comprising a step of contacting the polypeptide or a pharmacologically acceptable salt thereof of (1) with histone or a pentraxin 3 N-terminal domain-binding fragment thereof of (2), wherein the continuous partial sequence of (ii) comprises any of the following regions:
(1) a region consisting of the 1st - 50th amino acids of the amino acid sequence shown in SEQ ID NO: 3,
(2) a region consisting of the 38th - 87th amino acids of the amino acid sequence shown in SEQ ID NO: 3,
(3) a region consisting of the 75th - 124th amino acids of the amino acid sequence shown in SEQ ID NO: 3, and
(4) a region consisting of the 112th - 161st amino acids of the amino acid sequence shown in SEQ ID NO: 3, and
wherein the length of the polypeptide is not more than 100 amino acids.

9. The production method according to claim 8, wherein the polypeptide complex is a polypeptide aggregate.

## Patentansprüche

1. Polypeptid, umfassend:
(ii) eine zusammenhängende partielle Sequenz der in SEQ ID Nr. 3 gezeigten Aminosäuresequenz mit einer Länge von nicht weniger als 8 Aminosäuren; oder
(iii) eine Aminosäuresequenz, die nicht weniger als 70% Identität mit der Aminosäuresequenz von (ii) aufweist;
das Histon unter Bildung eines Polypeptidaggregats binden kann, oder ein pharmakologisch annehmbares Salz davon zur Verwendung zur Behandlung oder Prävention des systemischen inflammatorischen Response-Syndroms, wobei die zusammenhängende partielle Sequenz von (ii) einen der folgenden Bereiche umfasst:
(1) einen Bereich, der aus der 1. bis 50. Aminosäure der in SEQ ID Nr. 3 gezeigten Aminosäuresequenz besteht;
(2) einen Bereich, der aus der 38. bis 87. Aminosäure der in SEQ ID Nr. 3 gezeigten Aminosäuresequenz besteht;
(3) einen Bereich, der aus der 75. bis 124. Aminosäure der in SEQ ID Nr. 3 gezeigten Aminosäuresequenz besteht; und
(4) einen Bereich, der aus der 112. bis 161. Aminosäure der in SEQ ID Nr. 3 gezeigten Aminosäuresequenz besteht, und
wobei die Länge des Polypeptids nicht mehr als 100 Aminosäuren beträgt.

2. Verwendung eines Polypeptids, umfassend:
(ii) eine zusammenhängende partielle Sequenz der in SEQ ID Nr. 3 gezeigten Aminosäuresequenz mit einer Länge von nicht weniger als 8 Aminosäuren; oder
(iii) eine Aminosäuresequenz, die nicht weniger als 70% Identität mit der Aminosäuresequenz von (ii) aufweist;
das Histon unter Bildung eines Polypeptidaggregats binden kann, oder eines pharmakologisch annehmbaren Salzes davon zum Quantifizieren von Histon in vitro,
wobei die zusammenhängende partielle Sequenz von (ii) einen der folgenden Bereiche umfasst:
(1) einen Bereich, der aus der 1. bis 50. Aminosäure der in SEQ ID Nr. 3 gezeigten Aminosäuresequenz besteht;
(2) einen Bereich, der aus der 38. bis 87. Aminosäure der in SEQ ID Nr. 3 gezeigten Aminosäuresequenz besteht;
(3) einen Bereich, der aus der 75. bis 124. Aminosäure der in SEQ ID Nr. 3 gezeigten Aminosäuresequenz besteht; und
(4) einen Bereich, der aus der 112. bis 161. Aminosäure der in SEQ ID Nr. 3 gezeigten Aminosäuresequenz besteht, und
wobei die Länge des Polypeptids nicht mehr als 100 Aminosäuren beträgt.

3. Verwendung gemäß Anspruch 2, wobei das Polypeptid oder sein pharmakologisch annehmbares Salz auf einem Festphasenträger immobilisiert ist.

4. Verfahren zum Quantifizieren von Histon, umfassend
(1) einen Schritt des In-Kontakt-Bringens eines Polypeptids, umfassend:
(ii) eine zusammenhängende partielle Sequenz der in SEQ ID Nr. 3 gezeigten Aminosäuresequenz mit einer Länge von nicht weniger als 8 Aminosäuren; oder
(iii) eine Aminosäuresequenz, die nicht weniger als 70% Identität mit der Aminosäuresequenz von (ii) aufweist;
das Histon unter Bildung eines Polypeptidaggregats binden kann, oder eines pharmakologisch annehmbaren Salzes davon mit einer histonhaltigen Probe unter Bildung eines Polypeptidaggregats, das das Polypeptid oder sein pharmakologisch annehmbares Salz und Histon umfasst; und
(2) einen Schritt des Quantifizierens des in Schritt (1) erhaltenen Polypeptidaggregats,
wobei die zusammenhängende partielle Sequenz von (ii) einen der folgenden Bereiche umfasst:
(1) einen Bereich, der aus der 1. bis 50. Aminosäure der in SEQ ID Nr. 3 gezeigten Aminosäuresequenz besteht;
(2) einen Bereich, der aus der 38. bis 87. Aminosäure der in SEQ ID Nr. 3 gezeigten Aminosäuresequenz besteht;
(3) einen Bereich, der aus der 75. bis 124. Aminosäure der in SEQ ID Nr. 3 gezeigten Aminosäuresequenz besteht; und
(4) einen Bereich, der aus der 112. bis 161. Aminosäure der in SEQ ID Nr. 3 gezeigten Aminosäuresequenz besteht, und
wobei die Länge des Polypeptids nicht mehr als 100 Aminosäuren beträgt.

5. Verfahren gemäß Anspruch 4, wobei das in dem Polypeptidaggregat enthaltene Histon in Schritt (2) durch ein immunologisches Verfahren quantifiziert wird, indem man einen Antikörper verwendet, der Histon spezifisch erkennt.

6. Polypeptidkomplex, umfassend
(1) ein Polypeptid, umfassend:
(ii) eine zusammenhängende partielle Sequenz der in SEQ ID Nr. 3 gezeigten Aminosäuresequenz mit einer Länge von nicht weniger als 8 Aminosäuren; oder
(iii) eine Aminosäuresequenz, die nicht weniger als 70% Identität mit der Aminosäuresequenz von (ii) aufweist;
das Histon unter Bildung eines Polypeptidaggregats binden kann, oder ein pharmakologisch annehmbares Salz davon und
(2) Histon oder ein die N-terminale Domäne von Pentraxin 3 bindendes Fragment davon;
wobei die zusammenhängende partielle Sequenz von (ii) einen der folgenden Bereiche umfasst:
(1) einen Bereich, der aus der 1. bis 50. Aminosäure der in SEQ ID Nr. 3 gezeigten Aminosäuresequenz besteht;
(2) einen Bereich, der aus der 38. bis 87. Aminosäure der in SEQ ID Nr. 3 gezeigten Aminosäuresequenz besteht;
(3) einen Bereich, der aus der 75. bis 124. Aminosäure der in SEQ ID Nr. 3 gezeigten Aminosäuresequenz besteht; und
(4) einen Bereich, der aus der 112. bis 161. Aminosäure der in SEQ ID Nr. 3 gezeigten Aminosäuresequenz besteht, und
wobei die Länge des Polypeptids nicht mehr als 100 Aminosäuren beträgt.

7. Polypeptidkomplex gemäß Anspruch 6, der ein Polypeptidaggregat ist.

8. Verfahren zur Herstellung eines Polypeptidkomplexes, umfassend
(1) ein Polypeptid, umfassend:
(ii) eine zusammenhängende partielle Sequenz der in SEQ ID Nr. 3 gezeigten Aminosäuresequenz mit einer Länge von nicht weniger als 8 Aminosäuren; oder
(iii) eine Aminosäuresequenz, die nicht weniger als 70% Identität mit der Aminosäuresequenz von (ii) aufweist;
das Histon unter Bildung eines Polypeptidaggregats binden kann, oder ein pharmakologisch annehmbares Salz davon und
(2) Histon oder ein die N-terminale Domäne von Pentraxin 3 bindendes Fragment davon;
umfassend einen Schritt des In-Kontakt-Bringens des Polypeptids oder seines pharmakologisch annehmbaren Salzes von (1) mit Histon oder einem die N-terminale Domäne von Pentraxin 3 bindenden Fragment davon (2);
wobei die zusammenhängende partielle Sequenz von (ii) einen der folgenden Bereiche umfasst:
(1) einen Bereich, der aus der 1. bis 50. Aminosäure der in SEQ ID Nr. 3 gezeigten Aminosäuresequenz besteht;
(2) einen Bereich, der aus der 38. bis 87. Aminosäure der in SEQ ID Nr. 3 gezeigten Aminosäuresequenz besteht;
(3) einen Bereich, der aus der 75. bis 124. Aminosäure der in SEQ ID Nr. 3 gezeigten Aminosäuresequenz besteht; und
(4) einen Bereich, der aus der 112. bis 161. Aminosäure der in SEQ ID Nr. 3 gezeigten Aminosäuresequenz besteht, und
wobei die Länge des Polypeptids nicht mehr als 100 Aminosäuren beträgt.

9. Herstellungsverfahren gemäß Anspruch 8, wobei der Polypeptidkomplex ein Polypeptidaggregat ist.

## Revendications

1. Polypeptide comprenant
(ii) une séquence partielle continue de la séquence d'acides aminés représentée par SEQ ID NO : 3 ayant une longueur de pas moins de 8 acides aminés ; ou
(iii) une séquence d'acides aminés ne présentant pas moins de 70 % d'identité avec la séquence d'acides aminés de (ii) ;
qui est capable de se lier à des histones pour former un agrégat polypeptidique, ou l'un de ses sels pharmacologiquement acceptables, destiné à être utilisé pour le traitement ou la prévention du syndrome de réponse inflammatoire systémique, dans lequel la séquence partielle continue de (ii) comprend l'une quelconque des régions suivantes :
(1) une région constituée des 1er au 50ème acides aminés de la séquence d'acides aminés représentée par SEQ ID NO : 3,
(2) une région constituée des 38ème au 87ème acides aminés de la séquence d'acides aminés représentée par SEQ ID NO : 3,
(3) une région constituée des 75ème au 124ème acides aminés de la séquence d'acides aminés représentée par SEQ ID NO : 3, et
(4) une région constituée des 112ème au 161ème acides aminés de la séquence d'acides aminés représentée par SEQ ID NO : 3, et
dans lequel la longueur du polypeptide n'est pas de plus de 100 acides aminés.

2. Utilisation d'un polypeptide comprenant
(ii) une séquence partielle continue de la séquence d'acides aminés représentée par SEQ ID NO : 3 ayant une longueur de pas moins de 8 acides aminés ; ou
(iii) une séquence d'acides aminés ne présentant pas moins de 70 % d'identité avec la séquence d'acides aminés de (ii) ;
qui est capable de se lier à des histones pour former un agrégat polypeptidique, ou l'un de ses sels pharmacologiquement acceptables, pour quantifier des histones *in vitro,*
dans lequel la séquence partielle continue de (ii) comprend l'une quelconque des régions suivantes :
(1) une région constituée des 1er au 50ème acides aminés de la séquence d'acides aminés représentée par SEQ ID NO : 3,
(2) une région constituée des 38ème au 87ème acides aminés de la séquence d'acides aminés représentée par SEQ ID NO : 3,
(3) une région constituée des 75ème au 124ème acides aminés de la séquence d'acides aminés représentée par SEQ ID NO : 3, et
(4) une région constituée des 112ème au 161ème acides aminés de la séquence d'acides aminés représentée par SEQ ID NO : 3, et
dans lequel la longueur du polypeptide n'est pas de plus de 100 acides aminés.

3. Utilisation selon la revendication 2, dans laquelle le polypeptide ou l'un de ses sels pharmacologiquement acceptables est immobilisé sur un support en phase solide.

4. Procédé de quantification des histones, comprenant
(1) une étape de mise en contact d'un polypeptide comprenant
(ii) une séquence partielle continue de la séquence d'acides aminés représentée par SEQ ID NO : 3 ayant une longueur de pas moins de 8 acides aminés ; ou
(iii) une séquence d'acides aminés ne présentant pas moins de 70 % d'identité avec la séquence d'acides aminés de (ii) ;
qui est capable de se lier à des histones pour former un agrégat polypeptidique, ou l'un de ses sels pharmacologiquement acceptables, avec un échantillon contenant des histones pour former un agrégat polypeptidique comprenant le polypeptide ou l'un de ses sels pharmacologiquement acceptables et des histones, et
(2) une étape de quantification de l'agrégat polypeptidique obtenu dans l'étape (1),
dans lequel la séquence partielle continue de (ii) comprend l'une quelconque des régions suivantes :
(1) une région constituée des 1er au 50ème acides aminés de la séquence d'acides aminés représentée par SEQ ID NO : 3,
(2) une région constituée des 38ème au 87ème acides aminés de la séquence d'acides aminés représentée par SEQ ID NO : 3,
(3) une région constituée des 75ème au 124ème acides aminés de la séquence d'acides aminés représentée par SEQ ID NO : 3, et
(4) une région constituée des 112ème au 161ème acides aminés de la séquence d'acides aminés représentée par SEQ ID NO : 3, et
dans lequel la longueur du polypeptide n'est pas de plus de 100 acides aminés.

5. Procédé selon la revendication 4, dans lequel les histones contenues dans l'agrégat polypeptidique sont quantifiées dans l'étape (2) par un procédé immunologique en utilisant un anticorps qui reconnaît spécifiquement les histones.

6. Complexe polypeptidique comprenant
(1) un polypeptide comprenant
(ii) une séquence partielle continue de la séquence d'acides aminés représentée par SEQ ID NO : 3 ayant une longueur de pas moins de 8 acides aminés ; ou
(iii) une séquence d'acides aminés ne présentant pas moins de 70 % d'identité avec la séquence d'acides aminés de (ii) ;
qui est capable de se lier à des histones pour former un agrégat polypeptidique, ou l'un de ses sels pharmacologiquement acceptables, et
(2) des histones ou l'un de leurs fragments de liaison du domaine N-terminal de la pentraxine 3,
dans lequel la séquence partielle continue de (ii) comprend l'une quelconque des régions suivantes :
(1) une région constituée des 1er au 50ème acides aminés de la séquence d'acides aminés représentée par SEQ ID NO : 3,
(2) une région constituée des 38ème au 87ème acides aminés de la séquence d'acides aminés représentée par SEQ ID NO : 3,
(3) une région constituée des 75ème au 124ème acides aminés de la séquence d'acides aminés représentée par SEQ ID NO : 3, et
(4) une région constituée des 112ème au 161ème acides aminés de la séquence d'acides aminés représentée par SEQ ID NO : 3, et
dans lequel la longueur du polypeptide n'est pas de plus de 100 acides aminés.

7. Complexe polypeptidique selon la revendication 6, qui est un agrégat polypeptidique.

8. Procédé de production d'un complexe polypeptidique comprenant
(1) un polypeptide comprenant
(ii) une séquence partielle continue de la séquence d'acides aminés représentée par SEQ ID NO : 3 ayant une longueur de pas moins de 8 acides aminés ; ou
(iii) une séquence d'acides aminés ne présentant pas moins de 70 % d'identité avec la séquence d'acides aminés de (ii) ;
qui est capable de se lier à des histones pour former un agrégat polypeptidique, ou l'un de ses sels pharmacologiquement acceptables, et
(2) des histones ou l'un de leurs fragments de liaison du domaine N-terminal de la pentraxine 3, comprenant une étape de mise en contact du polypeptide ou de l'un de ses sels pharmacologiquement acceptables de (1) avec des histones ou l'un de leurs fragments de liaison du domaine N-terminal de la pentraxine 3 de (2),
dans lequel la séquence partielle continue de (ii) comprend l'une quelconque des régions suivantes :
(1) une région constituée des 1er au 50ème acides aminés de la séquence d'acides aminés représentée par SEQ ID NO : 3,
(2) une région constituée des 38ème au 87ème acides aminés de la séquence d'acides aminés représentée par SEQ ID NO : 3,
(3) une région constituée des 75ème au 124ème acides aminés de la séquence d'acides aminés représentée par SEQ ID NO : 3, et
(4) une région constituée des 112ème au 161ème acides aminés de la séquence d'acides aminés représentée par SEQ ID NO : 3, et
dans lequel la longueur du polypeptide n'est pas de plus de 100 acides aminés.

9. Procédé de production selon la revendication 8, dans lequel le complexe polypeptidique est un agrégat polypeptidique.
